# EUROPEAN PATENT APPLICATION

(11) **EP 4 790 142 A1**
(43) Date of publication of application: **12.08.2026**
(21) Application number: 24878369.8
(22) Date of filing: 19.03.2024
(51) Int. Cl.: C12P 13/00

(54) **METHOD FOR PREPARING HYDRAZINE COMPOUND**

(30) Priority: 16.10.2023 CN 202311335104; 08.02.2024 CN 202410177669
(71) Applicant: Jilin Asymchem Pharmaceuticals Co., Ltd., Dunhua, Yanbian, Jilin 133710 (CN); Tianjin Asymchem Biotechnology Co., Ltd., Tianjin 300457 (CN)
(72) Inventor: HONG, Hao, Morrisville, North Carolina 27560 (US); JAMES, Gage, Morrisville, North Carolina 27560 (US); ZHANG, Na, Tianjin 300457 (CN); JIAO, Xuecheng, Tianjin 300457 (CN); LI, Yanjun, Tianjin 300457 (CN); HUANG, Aiping, Tianjin 300457 (CN); SHEN, Peili, Tianjin 300457 (CN); SHI, Chengcheng, Tianjin 300457 (CN); MA, Tianjiao, Tianjin 300457 (CN)
(74) Representative: HGF
(86) International application number: PCT/CN2024/082521
(87) International publication number: WO 2025/081713

(57) **Abstract**

A method for preparing a hydrazine compound. The method includes: using an imine reductase to catalyze a reduction reaction between a substrate ketone compound as shown in formula I or formula III and a substrate hydrazine compound as shown in formula II, to obtain a product hydrazine compound. The imine reductase is used to perform the synthesis of the hydrazine compound, the synthesis process is simple and economical, various hydrazine compounds can be directly synthesized, and fewer substituent groups are present of the N atoms of the resulting hydrazine compounds. In addition, the method can be used for industrial scale-up, is low in cost and high in purity, and realizes genuine green chemistry.

## Description

This application is based upon and claims priority to Chinese Patent Application No. 202311335104.3 filed on October 16, 2023 and Chinese Patent Application No. 202410177669.1 filed on February 8, 2024, the disclosures of which are hereby incorporated by reference in their entirety.

### Technical Field

The present invention relates to the technical field of enzyme-catalyzed reactions, and specifically to a method for preparing a hydrazine compound.

### Background

Chiral hydrazine compounds, acylhydrazine, aromatic hydrazine, and other hydrazine compounds are extremely important compounds with widespread applications in the fields of biology, agriculture, and dyes. Furthermore, they also serve as key intermediates in the synthesis of many heterocyclic compounds. Some compounds containing hydrazine structures have potential pharmacological activity, and some of these are used clinically as Active Pharmaceutical Ingredients (APIs).

Patent applications (WO 2013083606A1 and US2009286812A1) reported a method for synthesizing a hydrazine compound by reacting ketone with hydrazine hydrate, followed by the addition of sodium borohydride. A patent application (WO 2012069948A1) reported the reaction of ketone and hydrazine hydrate in the presence of platinum-carbon. A study published in Angew. Chem., Int. Ed. 2019,58,15767 reported a method for generating a hydrazine compound by reducing hydrazone catalyzed by cobalt and zinc. Angew. Chem., Int. Ed. 2015,54,5112 reported a method for generating a hydrazine compound by reducing hydrazone catalyzed by nickel. ACS Catal. 2015,5,6086-6089 reported a method for generating a hydrazine compound from ketone and hydrazine catalyzed by palladium trifluoroacetate.

Methods for generating a hydrazine compound by using an enzyme to catalyze a reaction between a ketone compound and the hydrazine compound have not been reported yet. Moreover, in the related art, metal-catalyzed methods may have metal residues, are ligand-dependent, and generate hydrazine compounds carrying protective groups such as aromatic groups and benzoyl groups, posing challenges for subsequent reactions. Enzyme-catalyzed reactions are typically conducted under mild conditions and produce fewer by-products with lower impurity levels. Therefore, it is very important to provide a method for preparing a hydrazine compound through an enzyme-catalyzed reaction.

### Summary

A main objective of the present invention is to provide a method for preparing a hydrazine compound, so as to solve the problem of interference with subsequent application effects due to excessive substituents on an N atom of a hydrazine compound obtained by an existing method.

In order to implement the above objective, an aspect of the present invention provides a method for preparing a hydrazine compound. The method includes: an imine reductase is used to catalyze a reduction reaction between a substrate ketone compound shown in formula I or formula III and a substrate hydrazine compound shown in formula II, to obtain a product hydrazine compound. 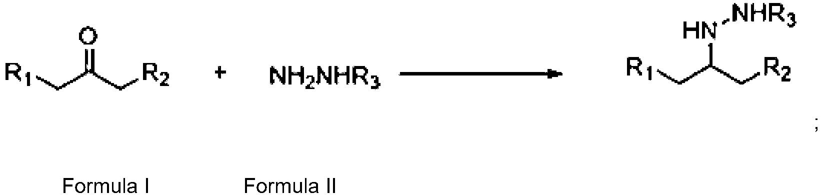 or 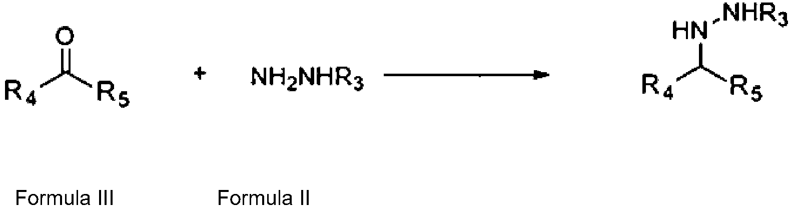 wherein R₁ and R₂ are each independently selected from hydrogen, cyano, , _{,} pyridine, furan, 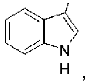 a C₁-C₄ alkyl, a C₅-C₁₀ cycloalkyl, or a C₅-C₁₀ substituted or unsubstituted heterocycloalkyl; or R₁ and R₂ are linked to form a C₃-C₈ substituted or unsubstituted cycloalkyl, a C₃-C₈ substituted or unsubstituted heterocycloalkyl, or a C₉-C₁₅ substituted or unsubstituted benzocycloalkyl; R₄ and R₅ are each independently selected from trifluoromethyl, cyano, arylmethyl, methoxy, substituted or unsubstituted phenyl, a C₁-C₄ alkyl, a C₅-C₁₀ cycloalkyl, a C₅-C₁₀ substituted or unsubstituted heterocycloalkyl, 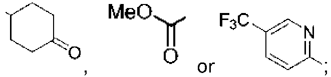 when the cycloalkyl, phenyl, or benzocycloalkyl is substituted, one or more H atoms of the cycloalkyl or benzocycloalkyl are optionally substituted with a halogen atom or an alkyl; one or more C atoms of the heterocycloalkyl are optionally substituted with N, O, P, or S atom; when the heterocycloalkyl is substituted, one or more H atoms of the heterocycloalkyl are optionally substituted with a halogen atom, benzyl, tert-butoxycarbonyl, or an alkyl; R₃ is selected from hydrogen, a C₁-C₄ alkyl, a C₆-C₁₀ aryl, a C₁-C₄ hydroxyalkyl, substituted or unsubstituted C₁-C₄ acyl, substituted or unsubstituted C₆-C₁₀ arylmethyl, tert-butoxycarbonyl, benzyloxycarbonyl, or 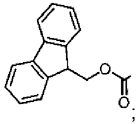 and when the acyl or arylmethyl is substituted, one or more H atoms of the acyl or arylmethyl are optionally substituted with a halogen atom, phenyl, or an alkyl.

Further, the imine reductase is selected from any one of the imine reductases shown in Table 1.

Further, R₁ and R₂ in the substrate ketone compound are each independently selected from hydrogen, cyano, , pyridine, furan, 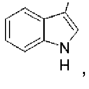 , a C₁-C₄ alkyl, a C₅-C₆ cycloalkyl, or a C₅-C₆ substituted or unsubstituted heterocycloalkyl; or R₁ and R₂ are linked to form a C₅-C₆ substituted or unsubstituted cycloalkyl, a C₅-C₆ substituted or unsubstituted heterocycloalkyl, or a C₉-C₁₃ substituted or unsubstituted benzocycloalkyl; R₄ and R₅ are each independently selected from trifluoromethyl, cyano, arylmethyl, methoxy, substituted or unsubstituted phenyl, a C₁-C₄ alkyl, a C₅-C₆ cycloalkyl, a C₅-C₆ substituted or unsubstituted heterocycloalkyl, 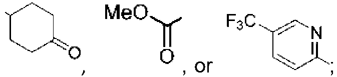 when the cycloalkyl, phenyl, or benzocycloalkyl is substituted, one or more H atoms of the cycloalkyl or benzocycloalkyl are optionally substituted with F atom, Cl atom, or methyl; one or more C atoms of the heterocycloalkyl are optionally substituted with N or O atoms; and when the heterocycloalkyl is substituted, one or more H atoms of the heterocycloalkyl are optionally substituted with Cl atom, F atom, benzyl, tert-butoxycarbonyl, or methyl.

Further, R₃ in the substrate hydrazine compound is selected from hydrogen, a C₁-C₂ alkyl, a C₆ aryl, a C₁-C₂ hydroxyalkyl, benzyl, tert-butoxycarbonyl, benzyloxycarbonyl, 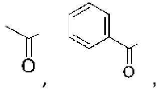 or 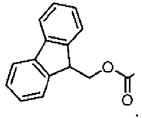 Further, the substrate ketone compound includes any one of the following: 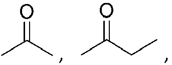 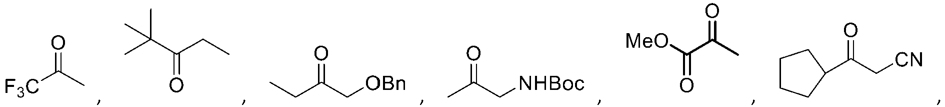 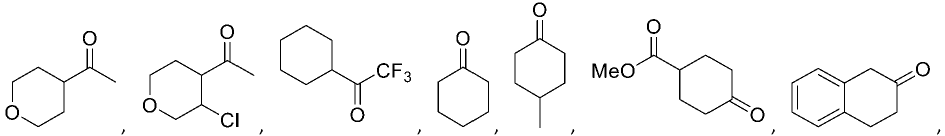 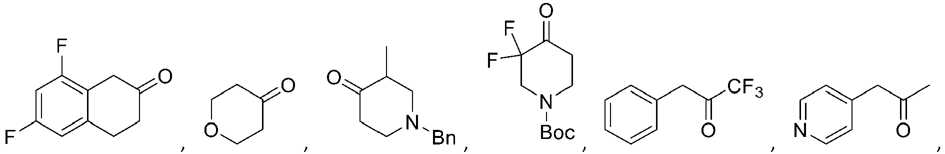 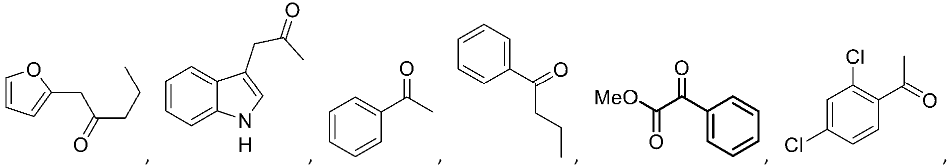 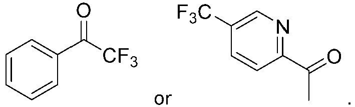 Further, the substrate hydrazine compound includes any one of the following: NH₂NH₂• H₂O, NH₂NHBoc, NH₂NHCbz, NH₂NH₂Bn,

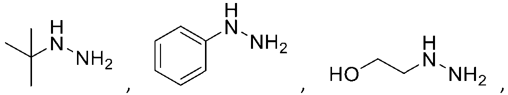

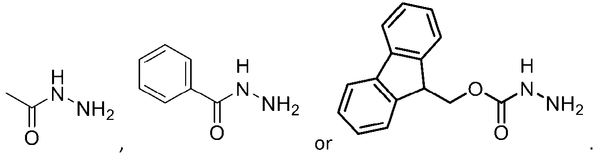

Further, in a reaction system for a reduction reaction, a molar ratio of the substrate ketone compound to the substrate hydrazine compound is 10-100:50-200.

Further, a mass ratio of the substrate ketone compound to the imine reductase is 1:1-10.

Further, the reaction system for the reduction reaction further includes a glucose dehydrogenase, D-glucose, nicotinamide adenine dinucleotide, nicotinamide adenine dinucleotide phosphate, and a buffer.

By using the technical solution of the present invention, the present invention uses the substrate with a specific structure to perform synthesis of the hydrazine compound catalyzed by the imine reductase, the synthesis process is simple and economical, various hydrazine compounds can be directly synthesized, and fewer substituents are present on an N atom of the product hydrazine compound. Moreover, the method can be well used for industrial scale-up, is low in cost and high in purity, and realizes genuine green chemistry.

### Detailed Description of the Embodiments

It is to be noted that the embodiments in the present invention and the features in the embodiments may be combined with one another without conflict. The present invention will be described below in detail with reference to the embodiments.

As mentioned in the Background, in the related art, the synthesis of hydrazine compounds is typically performed by using metal-catalyzed methods including reactions between ketone compounds and hydrazine compounds. The synthesis methods may have metal residues, are ligand-dependent, and generate hydrazine compounds carrying protective groups such as aromatic groups and benzoyl groups, posing challenges for subsequent reactions. An enzyme-catalyzed reaction is mild in condition and does not introduce significant amounts of impurities. Furthermore, an N atom in the generated hydrazine compound has no substituents or only one substituent, facilitating the subsequent use of the compound. Therefore, the present invention provides a method for synthesizing a hydrazine compound using an enzyme-catalyzed reaction, so as to achieve the purpose of efficient and industrial-scale production of hydrazine compounds.

In a first typical implementation of the present invention, a method for preparing a hydrazine compound is provided. The method includes: imine reductase is used to catalyze a reduction reaction between a substrate ketone compound shown in formula I or formula III and a substrate hydrazine compound shown in formula II, to obtain a product hydrazine compound. 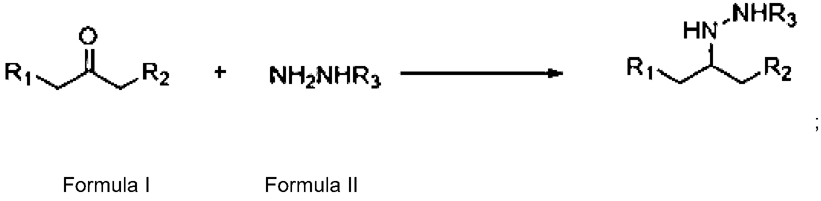 or 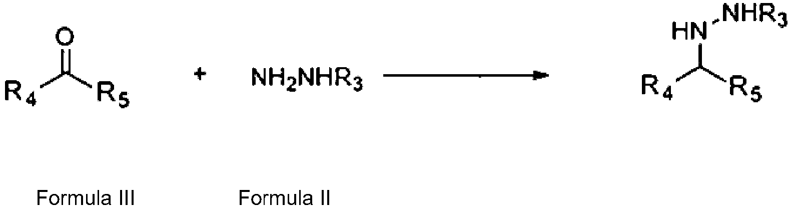 wherein R₁ and R₂ are each independently selected from hydrogen, cyano, , pyridine, furan, 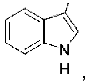 a C₁-C₄ alkyl, a C₅-C₁₀ cycloalkyl, or a C₅-C₁₀ substituted or unsubstituted heterocycloalkyl; or R₁ and R₂ are linked to form a C₃-C₈ substituted or unsubstituted cycloalkyl, a C₃-C₈ substituted or unsubstituted heterocycloalkyl, or a C₉-C₁₅ substituted or unsubstituted benzocycloalkyl; R₄ and R₅ are each independently selected from trifluoromethyl, cyano, arylmethyl, methoxy, substituted or unsubstituted phenyl, a C₁-C₄ alkyl, a C₅-C₁₀ cycloalkyl, a C₅-C₁₀ substituted or unsubstituted heterocycloalkyl, 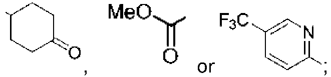 when the cycloalkyl, phenyl, or benzocycloalkyl is substituted, one or more H atoms of the cycloalkyl or benzocycloalkyl are optionally substituted with halogen atoms or alkyl; one or more C atoms of the heterocycloalkyl are optionally substituted with N, O, P, and S atom; when the heterocycloalkyl is substituted, one or more H atoms of the heterocycloalkyl are substituted with a halogen atom, benzyl, tert-butoxycarbonyl, or an alkyl; R₃ is selected from hydrogen, a C₁-C₄ alkyl, a C₆-C₁₀ aryl, a C₁-C₄ hydroxyalkyl, substituted or unsubstituted C₁-C₄ acyl, substituted or unsubstituted C₆-C₁₀ arylmethyl, tert-butoxycarbonyl, benzyloxycarbonyl, or 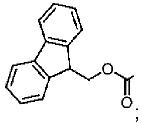 and when the acyl or arylmethyl is substituted, one or more H atoms of the acyl or arylmethyl are optionally substituted with a halogen atom, phenyl, or an alkyl.

Hydrazone is generated by the ketone compound and the hydrazine compound catalyzed by the imine reductase, the active hydrogen of NAD(P)H undergoes a nucleophilic addition to a C=N bond of the hydrazone, so as to generate nitrogen anion; and protons are then provided by surrounding positively charged amino acids or water molecules, to generate the hydrazine compound, so as to obtain the product hydrazine compound in which the N atom has no substituents or only one substituent. In a preferred embodiment, the imine reductase is an NAD(P)H-dependent enzyme. During the catalytic process of these enzymes, NAD(P)H is consumed to supply hydrogen, so as to be converted into NAD(P)⁺. Furthermore, in order to save costs, glucose is oxidized to corresponding gluconolactone, transferring electrons to the NAD(P)⁺, thereby allowing coenzyme to be recycled (referring to an equation shown below).

A series of imine reductase is screened by determining, as a screening condition, whether a catalytic reaction of a series of imine reductase in the generation of the hydrazine compound from the ketone compound and the hydrazine compound can catalyze the generation of the hydrazine compound.

The above enzymes are used for reaction, the hydrazone generated by the substrate ketone compound and the hydrazine compound is reduced to obtain the product hydrazine compound in which the required N atom has no substituents or only one substituent, such that the synthesis of the hydrazine compound can be performed simpler and more convenient to better perform subsequent application of the compound, and is more suitable for industrial-scale production.

Any imine reductase capable of undergoing the above reaction is applicable to the present invention. In a preferred embodiment, the imine reductase is selected from any one of imine reductase shown in Table 1.

**Table 1:**

| Number | Source | Serial number |
|---|---|---|
| 1 | Kribbella flavida DSM 17836 | WP_012921542.1 |
| 2 | Cupriavidus sp. HPC(L) | WP_023264430.1 |
| 3 | Nocardia cyriacigeorgica | WP_014349966.1 |
| 4 | Streptomyces sp. CNH287 | WP_027751652.1 |
| 5 | Streptomyces ipomoeae | WP_009330409.1 |
| 6 | Actinomadura oligospora | WP_026413575.1 |
| 7 | Mesorhizobium sp. L48C026A00 | WP_023799151.1 |
| 8 | Aeromonas veronii | WP_005354889.1 |
| 9 | Streptomyces chattanoogensis | A0A0N0XYF8_9ACTN |
| 10 | Cystobacter ferrugineus | A0A1L9AVJ5_9DELT |
| 11 | Eucalyptus grandis | A0A059CN57_EUCGR |
| 12 | Aspergillus terreus NIH2624 | XP_001217087 |
| 13 | Metagenomic | UPK83284.1 |
| 14 | Streptomyces noursei ATCC 11455 | ANZ18125.1 |
| 15 | Penicillium camemberti | CRL25037.1 |
| 16 | Madurella mycetomatis | KXX80955.1 |
| 17 | Thermothelomyces thermophilus ATCC 42464 | XM_003664535.1 |
| 18 | Nectria haematococca | XP_003047297.1 |
| 19 | Amycolatopsis regifaucium | WP_061987485.1 |
| 20 | Aspergillus lentulus | XP_033411594.1 |
| 21 | bacterium (compost metagenome) | PZN88780.1 |
| 22 | unclassified Micromonospora | WP_096761538.1 |
| 23 | Aspergillus udagawae | XP_043147525.1 |
| 24 | Mesorhizobium sp. L2C084A000 | WP_023809753.1 |
| 25 | Streptomyces sp. CNS615 | WP_026165099.1 |
| 26 | Salinispora pacifica | WP_026324618.1 |
| 27 | Rhizobium sullae | WP_027512056.1 |
| 28 | Mesorhizobium ciceri | WP_029347354.1 |
| 29 | Ensifer adhaerens | WP_038576230.1 |
| 30 | Labilithrix luteola | AKU97888.1 |
| 31 | Ensifer adhaerens | KOF13709.1 |
| 32 | Saccharothrix espanaensis | WP_015102218.1 |
| 33 | Leishmania major | XP_001683351.1 |
| 34 | Streptomyces thermolilacinus | wp_023587323.1 |
| 35 | Microbulbifer | WP_067084177.1 |
| 36 | Actinomadura rifamycini | WP_026403156.1 |
| 37 | Myxococcus stipitatus | WP_015347361.1 |
| 38 | Streptomyces spectabilis | PDB: 7OG3_A |
| 39 | Streptomyces | WP_003986432.1 |
| 40 | Variovorax beijingensis | WP_124961062.1 |
| 41 | Sinorhizobium sp. Sb3 | WP_063893400.1 |
| 42 | unclassified Streptomyces | WP_018515820.1 |
| 43 | Blastomyces gilchristii SLH14081 | XP_002626263.1 |
| 44 | Variovorax paradoxus | KPU97715.1 |
| 45 | Silvimonas sp. | WP_309327064.1 |
| 46 | Streptomyces aureocirculatus | WP_225882176.1 |
| 47 | Streptomyces sp. TOR3209 | WP_019324525.1 |
| 48 | Synechococcus elongatus PCC 6301 | BAD80427.1 |
| 49 | Streptomyces sp. ST1020 | WP_112474446.1 |
| 50 | Streptomyces albidoflavus | WP_010638508.1 |
| 51 | Allokutzneria albata | WP_030431272.1 |
| 52 | Mesorhizobium sp. 1M-11 | WP_054311034.1 |
| 53 | Sandaracinus amylolyticus | WP_075097693.1 |
| 54 | Rhizobium sp. LCM 4573 | WP_083347639.1 |
| 55 | Myxococcus fulvus | WP_074958336.1 |
| 56 | Ensifer adhaerens | WP_077961001.1 |
| 57 | Mesorhizobium mediterraneum | PAQ01898.1 |
| 58 | Ensifer sp. | WP_312362681.1 |
| 59 | Streptomyces | WP_015610874.1 |
| 60 | Streptomyces sp. GF3546 | AB747175.1 |
| 61 | Stackebrandtia nassauensis | WP_013019548.1 |
| 62 | Saccharothrix espanaensis | WP_015105194.1 |
| 63 | Amycolatopsis decaplanina | WP_007032738.1 |
| 64 | Streptomyces kanamyceticus | WP_107099103.1 |
| 65 | Yersinia enterocolitica | WP_005168671.1 |
| 66 | Pseudomonas aeruginosa | WP_023094438.1 |
| 67 | Bacillus cereus | WP_000841638.1 |
| 68 | Nocardiopsis halophila | WP_017538739.1 |
| 69 | Amycolatopsis keratiniphila | WP_016332075.1 |
| 70 | Streptomyces | WP_006374254.1 |
| 71 | Paenibacillus elgii | WP_010497949.1 |
| 72 | Streptosporangium roseum | WP_012887675.1 |
| 73 | Paenibacillus lactis | WP_007128231.1 |
| 74 | Streptomyces aurantiacus | WP_016644884.1 |
| 75 | Aspergillus oryzae | 5G6S_E |
| 76 | Aeromonas veronii | WP_005335883.1 |
| 77 | Streptomyces tsukubaensis | WP_006347397.1 |
| 78 | Nocardia brasiliensis | WP_014988976.1 |
| 79 | Streptomyces viridochromogenes | WP_004001465.1 |
| 80 | Mycobacterium smegmatis | WP_011731218.1 |
| 81 | Micromonospora | WP_013733165.1 |
| 82 | Arthrobacter sp. | BAA08145.1 |
| 83 | Pecten maximus | 3C7A_A |
| 84 | Micromonospora tulbaghiae | WP_091423753.1 |
| 85 | Streptomyces cyaneogriseus | WP_063856803.1 |
| 86 | Streptomyces sp. TSRI0107 | WP_073938678.1 |
| 87 | Streptomyces bobili | WP_086772432.1 |
| 88 | Streptomyces ossamyceticus | WP_079058916.1 |
| 89 | Streptomyces sp. NRRL S-340 | WP_037855546.1 |
| 90 | Streptomyces sp. SA15 | WP_095755701.1 |
| 91 | Saccharothrix syringae | WP_033428623.1 |
| 92 | Saccharothrix sp. NRRL B-16348 | WP_053718467.1 |
| 93 | Amycolatopsis alba | WP_020635633.1 |
| 94 | Amycolatopsis nigrescens | WP_020666256.1 |
| 95 | Yersinia enterocolitica subsp. enterocolitica 8081 | PDB: 4GMG_A |
| 96 | Streptomyces avidinii | PDB: 6ESU_A |
| 97 | Saccharomonospora glauca | WP_005464539.1 |
| 98 | Amycolatopsis keratiniphila | WP_016333210.1 |
| 99 | Streptomyces microflavus | WP_015611605.1 |
| 100 | Actinomadura madurae | WP_021595532.1 |
| 101 | Streptomyces pristinaespiralis | WP_005316299.1 |
| 102 | Frankia sp. EAN1pec | ABW15942.1 |
| 103 | Saccharomonospora xinjiangensis | WP_006240684.1 |
| 104 | Stachybotrys chartarum IBT 40288 | KFA74014.1 |
| 105 | Fusarium oligoseptatum | RSL78883.1 |
| 106 | Pseudogymnoascus sp. VKM F-4516 (FW-969) | KFY62601.1 |
| 107 | Exophiala aquamarina CBS 119918 | XP_013255118.1 |
| 108 | Lomentospora prolificans | PKS06169.1 |
| 109 | Microdochium bolleyi | KXJ84868.1 |
| 110 | Saccharopolyspora elongata | WP_132480472.1 |
| 111 | unclassified Mesorhizobium | WP_202364320.1 |
| 112 | Streptomyces netropsis | WP_184732442.1 |
| 113 | Streptomyces violaceusniger | GDY58834.1 |
| 114 | Actinokineospora iranica | WP_091451154.1 |
| 115 | Sinorhizobium fredii | WP_037435231.1 |
| 116 | Nonomuraea maritima | WP_090760597.1 |
| 117 | Actinomycetes | WP_009080768.1 |
| 118 | Streptoalloteichus hindustanus | WP_268844412.1 |
| 119 | Actinomadura rayongensis | WP_161102753.1 |
| 120 | Aspergillus bertholletiae | KAE8374192.1 |
| 121 | unclassified Ensifer | WP_090298514.1 |
| 122 | Streptoalloteichus hindustanus | WP_268844412.1 |
| 123 | Streptomyces buecherae | WP_187059892.1 |
| 124 | Saccharothrix saharensis | WP_141983583.1 |
| 125 | Mycolicibacterium tusciae | WP_083126537.1 |
| 126 | Streptomyces gardneri | WP_141302249.1 |
| 127 | Paenibacillus methanolicus | WP_148929187.1 |
| 128 | Streptomyces corynorhini | WP_114621863.1 |
| 129 | Nocardioides thalensis | WP_179669358.1 |
| 130 | Mesorhizobium sp. L48C026A00 | ESZ22675.1 |
| 131 | Paenibacillus methanolicus | WP_148930631.1 |
| 132 | Streptomyces buecherae | WP_187059892.1 |
| 133 | Streptomyces corynorhini | WP_114621863.1 |
| 134 | Saccharothrix saharensis | WP_141983583.1 |
| 135 | Paenibacillus methanolicus | WP_148929187.1 |
| 136 | Umezawaea tangerina | WP_106186768.1 |
| 137 | Streptomyces gardneri | WP_141302249.1 |
| 138 | Streptoalloteichus hindustanus | WP_268844412.1 |
| 139 | Chitinophaga eiseniae | WP_078667737.1 |
| 140 | Nocardioides thalensis | WP_179669358.1 |
| 141 | Saccharothrix saharensis | WP_141983583.1 |
| 142 | unclassified Gordonia (in: high G+C Gram-positive bacteria) | WP_064866657.1 |
| 143 | Corallococcus exercitus | WP_272491473.1 |
| 144 | Crateriforma conspicua | WP_146415321.1 |
| 145 | unclassified Ensifer | WP_090298514.1 |
| 146 | Lipingzhangella halophila | WP_184575048.1 |
| 147 | Streptomyces sp. SID8352 | MYU23206.1 |
| 148 | Phycisphaera sp. TMED9 | RPG18620.1 |
| 149 | Rhizobacter sp. Root1221 | WP_056657818.1 |
| 150 | Streptomyces luteolifulvus | WP_150950710.1 |
| 151 | Bailinhaonella thermotolerans | WP_119926963.1 |
| 152 | Bailinhaonella thermotolerans | WP_119927492.1 |
| 153 | unclassified Rhodococcus (in: high G+C Gram-positive bacteria) | WP_121872289.1 |
| 154 | Rhodophyticola porphyridii | RMA41460.1 |
| 155 | Gordonia asplenii | WP_170193380.1 |
| 156 | Chryseobacterium piperi | WP_034685408.1 |
| 157 | Streptomyces sp. BR123 | WP_179172528.1 |
| 158 | Bailinhaonella thermotolerans | WP_119929815.1 |
| 159 | Amycolatopsis azurea | WP_005159789.1 |
| 160 | Amycolatopsis azurea | WP_005159787.1 |
| 161 | Amycolatopsis azurea | WP_005152129.1 |
| 162 | Amycolatopsis decaplanina | WP_007029743.1 |
| 163 | Brevibacillus brevis | WP_106653702.1 |
| 164 | Myxococcus stipitatus | WP_015347789.1 |
| 165 | Saccharopolyspora erythraea | WP_009950787.1 |
| 166 | Kribbella flavida | WP_012918904.1 |
| 167 | Paenibacillus elgii | WP_127462484.1 |
| 168 | Paenibacillus elgii | WP_127459725.1 |
| 169 | Paenibacillus elgii | WP_127462485.1 |
| 170 | Paenibacillus lactis | WP_007131315.1 |
| 171 | Paenibacillus lactis | WP_007130043.1 |
| 172 | Paenibacillus lactis | WP_007131314.1 |
| 173 | Streptomyces | WP_037651136.1 |
| 174 | Streptomyces purpureus | WP_189202776.1 |
| 175 | Streptomyces viridochromogenes | WP_003988467.1 |
| 176 | Saccharothrix espanaensis | WP_015102111.1 |
| 177 | Streptomyces purpureus | WP_189205233.1 |
| 178 | Saccharothrix espanaensis DSM 44229 | CCH32106.1 |
| 179 | Saccharopolyspora erythraea | WP_009951565.1 |
| 180 | Streptomyces viridochromogenes | WP_003988471.1 |
| 181 | Streptomyces viridochromogenes | WP_039934401.1 |
| 182 | Streptomyces sp. SID8375 | MYX09070.1 |
| 183 | Stackebrandtia nassauensis | WP_013016842.1 |
| 184 | Stackebrandtia nassauensis | WP_013019549.1 |
| 185 | Mesorhizobium sp. WSM3626 | WP_027141806.1 |
| 186 | Amycolatopsis azurea | WP_005158512.1 |
| 187 | Monosporascus sp. GIB2 | RYP23847.1 |
| 188 | Kribbella flavida | WP_012923662.1 |
| 189 | Paenibacillus elgii | WP_063179310.1 |
| 190 | Paenibacillus elgii | WP_063181203.1 |
| 191 | Purpureocillium lilacinum | PWI71520.1 |
| 192 | Saccharopolyspora erythraea | WP_009949443.1 |
| 193 | Streptomyces kanamyceticus | WP_055554811.1 |
| 194 | Streptomyces kanamyceticus | WP_055549409.1 |
| 195 | Stackebrandtia nassauensis | WP_013019558.1 |
| 196 | Streptomyces purpureus | WP_019891836.1 |
| 197 | Purpureocillium lilacinum | XP_018179586.1 |
| 198 | Fusarium sp. AF-6 | RSL68789.1 |
| 199 | Amycolatopsis thermoflava | WP_027931121.1 |
| 200 | Mesorhizobium temperatum | WP_095494073.1 |
| 201 | Rhizobium sophorae | WP_077989474.1 |
| 202 | Sciscionella marina | WP_020496004.1 |
| 203 | Sinorhizobium medicae | WP_018011194.1 |
| 204 | Myceliophthora thermophila | XP_003664583.1 |
| 205 | Blastomyces dermatitidis ER-3 (Ajellomyces dermatitidis ER-3) | XP_045278909.1 |
| 206 | Myxococcus fulvus | AKF85151.1 |
| 207 | Cystobacter ferrugineus | WP_071905000.1 |
| 208 | Aspergillus oryzae | XP_001827659.1 |
| 209 | Aspergillus terreus | XP_001217087.1 |
| 210 | Actinoalloteichus hymeniacidonis | MBB5908345.1 |
| 211 | Streptomyces kanamyceticus | BAE95580.1 |
| 212 | [Torrubiella] hemipterigena | CEJ93364.1 |
| 213 | Tepidiphilus thermophilus | WP_055423924.1 |
| 214 | Thermobaculum terrenum | WP_012876437.1 |
| 215 | Thermopetrobacter sp. TC1 | WP_038033941.1 |
| 216 | Caldithrix abyssi DSM13497 | WP_006928738.1 |
| 217 | Thermosulfidibacter takaii | WP_068550104.1 |
| 218 | Thermanaerothrix daxensis | WP_083461621.1 |
| 219 | Xylella fastidiosa | WP_010893958.1 |
| 220 | Methylocaldum szegediense | WP_026609689.1 |
| 221 | Sulfuricella sp. T08 | WP_059418963.1 |
| 222 | Candidatus Reidiella endopervernicosa | WP_078484956.1 |
| 223 | Alteromonas sp. RW2A1 | WP_071979230.1 |
| 224 | Cycloclasticus sp. | PHR50282.1 |
| 225 | Moraxella sp. VT-16-12 | WP_089538611.1 |
| 226 | Leptomonas pyrrhocoris | XP_015662550.1 |
| 227 | Trypanosoma theileri | ORC83714.1 |
| 228 | Pseudonocardia thermophila | WP_073459042.1 |
| 229 | Prauserella marina | WP_091804541.1 |
| 230 | Streptoalloteichus hindustanus | SHE96216.1 |
| 231 | Kribbella catacumbae | WP_020388085.1 |
| 232 | Micromonospora echinaurantiaca | WP_088993565.1 |
| 233 | Nocardiopsis chromatogenes | WP_017622916.1 |
| 234 | Ensifer | WP_057221059.1 |
| 235 | Streptomyces iranensis | WP_044567941.1 |
| 236 | Amycolatopsis alba | WP_020635634.1 |
| 237 | Mesorhizobium sp. LSHC420B00 | WP_023720294.1 |
| 238 | Devosia sp. Root413D1 | WP_055997555.1 |
| 239 | Bosea sp. BIWAKO-01 | WP_069881969.1 |
| 240 | Rhizobium leguminosarum | WP_081250846.1 |
| 241 | Mesorhizobium sp. L2C084A000 | WP_081734918.1 |
| 242 | Chelatococcus | WP_082312585.1 |
| 243 | Rhizobium sp. LCM 4573 | WP_083347639.1 |
| 244 | Aminobacter aminovorans | WP_083948736.1 |
| 245 | Pseudorhodoplanes sinuspersici | WP_086088856.1 |
| 246 | Streptomyces scabiei | WP_086800502.1 |
| 247 | Vibrio gazogenes | WP_088133675.1 |
| 248 | Mycolicibacterium mageritense | WP_036431480.1 |
| 249 | Streptomyces toyocaensis | WP_037930878.1 |
| 250 | Streptomyces cyaneogriseus | WP_044382979.1 |
| 251 | Paenibacillus beijingensis | WP_045670610.1 |
| 252 | Streptomyces viridochromogenes | WP_004000144.1 |
| 253 | Streptomyces glaucescens | WP_043502029.1 |
| 254 | Streptomyces coelicoflavus ZG0656 | EHN79931.1 |
| 255 | Rhizobium sullae | WP_084606578.1 |
| 256 | Mesorhizobium | WP_084831592.1 |
| 257 | Ensifer | WP_034788492.1 |
| 258 | Kribbella flavida DSM 17836 | ADB32986.1 |
| 259 | Streptomyces viridochromogenesTue57 | ELS53044.1 |
| 260 | Glycomyces tenuis | WP_026930447.1 |
| 261 | Nitratireductor pacificus | WP_008598530.1 |
| 262 | Actinoalloteichus hymeniacidonis | AOS63607.1 |
| 263 | Amycolatopsis keratiniphila | WP_072027083.1 |
| 264 | Streptomyces argillaceus | SCO70303.1 |
| 265 | Allokutzneria albata | WP_030433268.1 |
| 266 | Amycolatopsis japonica | WP_038513254.1 |
| 267 | Amycolatopsis thermoflava | WP_027931120.1 |
| 268 | Burkholderia lata | WP_011349484.1 |
| 269 | Burkholderia sp. | WP_011882972.1 |
| 270 | Couchioplanes caeruleus | ROP32867.1 |
| 271 | Chelatococcus sambhunathii | WP_072249429.1 |
| 272 | Devosia geojensis | WP_046110744.1 |
| 273 | Draconibacterium orientale | WP_038562965.1 |
| 274 | Desulfosporosinus orientis | WP_014182981.1 |
| 275 | Blastomyces silverae | KLJ07305.1 |
| 276 | Emergomyces pasteurianus Ep9510 | OJD16557.1 |
| 277 | Isosphaera pallida | WP_013564402.1 |
| 278 | Jiangella alkaliphila | WP_046770251.1 |
| 279 | Kitasatospora cheerisanensis KCTC 2395 | KDN87324.1 |
| 280 | Kibdelosporangium phytohabitans | WP_054288132.1 |
| 281 | Granulicella mallensis | WP_014265743.1 |
| 282 | Microvirga pakistanensis | WP_134495755.1 |
| 283 | Aspergillus calidoustus | CEL10946.1 |
| 284 | Aspergillus udagawae | GIC90259.1 |
| 285 | Aspergillus bombycis | XP_022383494.1 |
| 286 | Aspergillus nidulans FGSC A4 | XP_681174.1 |
| 287 | Fonsecaea multimorphosa CBS 102226 | XP_016632757.1 |
| 288 | Fusarium oxysporum f. sp. melonis 26406 | EXK23898.1 |
| 289 | Eucalyptus grandis | KCW79631.1 |
| 290 | Picea sitchensis | ABK25898.1 |
| 291 | Arabis alpine | KFK29464.1 |
| 292 | Arabidopsis lyrata subsp. lyrata | XP_020872896.1 |
| 293 | Arabidopsis thaliana | NP_194641.1 |
| 294 | Brassica rapa | XP_009148011.1 |
| 295 | Corchorus capsularis | OMO74266.1 |
| 296 | Citrus clementine | XP_006451305.1 |
| 297 | Cucumis sativus | XP_004141022.2 |
| 298 | Daucus carota subsp. sativus | XP_017237888.1 |
| 299 | Eutrema salsugineum | XP_006393506.2 |
| 300 | Gossypium hirsutum | XP_016682550.1 |
| 301 | Jatropha curcas | KDP33798.1 |
| 302 | Lupinus angustifolius | OIV93407.1 |
| 303 | Morus notabilis | XP_010113158.1 |
| 304 | Homo sapiens | NP_115958.2 |
| 305 | Streptomyces purpureus | WP_019888617.1 |
| 306 | Streptomyces sp. PRh5 | WP_037958028.1 |

Any ketone compound substrate capable of undergoing the reduction reaction catalyzed by the above enzymes to produce the hydrazine compound is applicable to the present invention. In a preferred embodiment, R₁ and R₂ in the substrate ketone compound are each independently selected from hydrogen, cyano, 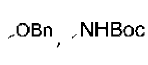 , pyridine, furan, 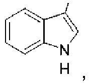 a C₁-C₄ alkyl, a C₅-C₆ cycloalkyl, or a C₅-C₆ substituted or unsubstituted heterocycloalkyl; or R₁ and R₂ are linked to form a C₅-C₆ substituted or unsubstituted cycloalkyl, a C₅-C₆ substituted or unsubstituted heterocycloalkyl, or a C₉-C₁₃ substituted or unsubstituted benzocycloalkyl; R₄ and R₅ are each independently selected from trifluoromethyl, cyano, arylmethyl, methoxy, substituted or unsubstituted phenyl, a C₁-C₄ alkyl, a C₅-C₆ cycloalkyl, a C₅-C₆ substituted or unsubstituted heterocycloalkyl, 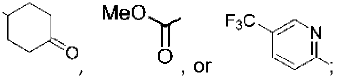 when the cycloalkyl, phenyl, or benzocycloalkyl is substituted, one or more H atoms of the cycloalkyl or benzocycloalkyl are optionally substituted with F atom, Cl atom, or methyl; one or more C atoms of the heterocycloalkyl are optionally substituted with N or O atoms; and when the heterocycloalkyl is substituted, one or more H atoms of the heterocycloalkyl are optionally substituted with Cl atom, F atom, benzyl, tert-butoxycarbonyl, or methyl.

Any hydrazine compound substrate capable of undergoing the reduction reaction catalyzed by the above enzymes to produce the hydrazine compound is applicable to the present invention. In a preferred embodiment, R₃ in the substrate hydrazine compound is selected from hydrogen, a C₁-C₂ alkyl, a C₆ aryl, a C₁-C₂ hydroxyalkyl, a C₁-C₄ acyl, substituted or unsubstituted arylmethyl, tert-butoxycarbonyl, benzyloxycarbonyl, or

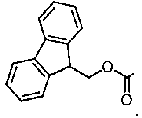

In a preferred embodiment, the substrate ketone compound includes any one of the following:

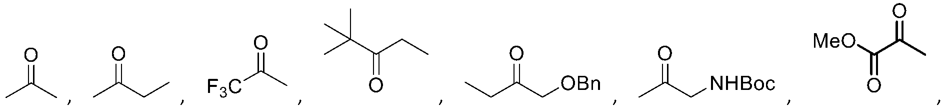

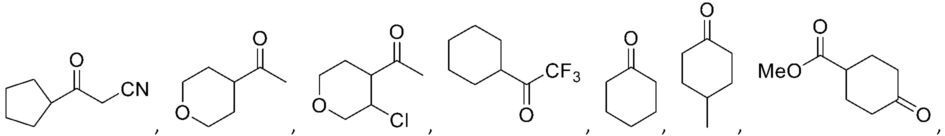

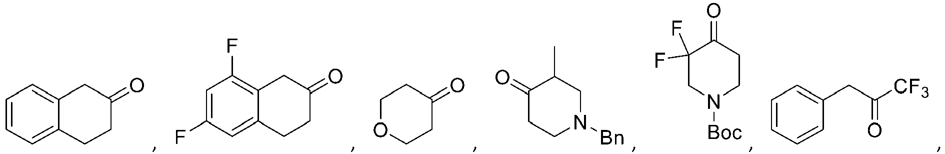

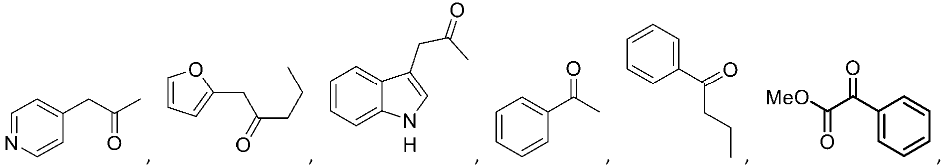

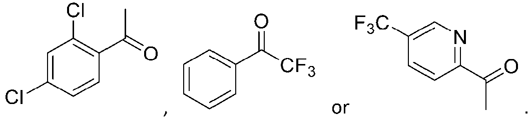

In a preferred embodiment, the substrate hydrazine compound includes any one of the following: NH₂NH₂·H₂O, NH₂NHBoc, NH₂NHCbz, NH₂NH₂Bn, NH₂NH₂•H₂O, NH₂NHBoc, NH₂NHCbz, NH₂NH₂Bn 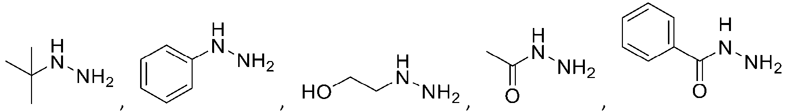 or

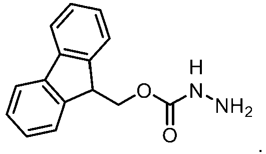

The addition amounts of any substrate and enzyme used in the synthesis of the hydrazine compound catalyzed by the imine reductase are all applicable to the present invention. From the perspective of improving reaction efficiency, in a preferred embodiment, in a reaction system for a reduction reaction, a molar ratio of the substrate ketone compound to the substrate hydrazine compound is 10-100:50-200; a mass ratio of the substrate ketone compound to the imine reductase is 1:1-10; and specifically, the mass ratio of the substrate ketone compound to the imine reductase is 1:1, 1:2, 1:3, 1:4, 1:5, 1:6, 1:7, 1:8, 1:9, or 1:10.

A chemical substance providing electrons is also required in the process of using the imine reductase to catalyze the substrate ketone compound and the substrate hydrazine compound to generate the product hydrazine compound in which the N atom has no substituents or only one substituent. Any chemical substance that can provide electrons is applicable to the present invention. In a preferred embodiment, the reaction system for the reduction reaction further includes glucose dehydrogenase, D-glucose, nicotinamide adenine dinucleotide, nicotinamide adenine dinucleotide phosphate, and a buffer. In a preferred embodiment, the buffer is Tris-HCl, a concentration of the Tris-HCl is 100 Mm, and the pH value of the Tris-HCl is 7.5-10.0.

The nicotinamide adenine dinucleotide and the nicotinamide adenine dinucleotide phosphate can provide reducing equivalents for the synthesis reaction of the hydrazine compound of the present invention. Moreover, cofactor regeneration is achieved by coupling with the glucose dehydrogenase-catalyzed oxidation of D-glucose to gluconolactone, thereby reducing the overall cost of the reaction, as shown in the following equation.

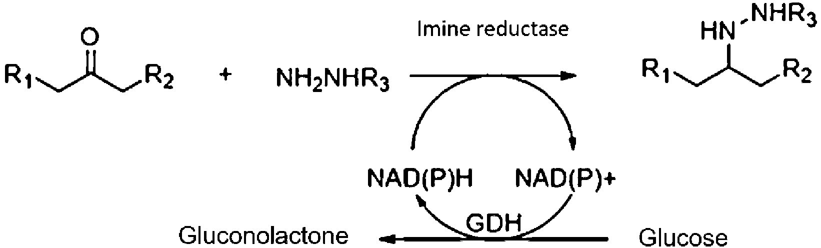

Any reaction condition conductive to the synthesis of the hydrazine compound is applicable to the present invention. From the perspective of reduction reaction efficiency and energy consumption, in a preferred embodiment, the time for the reduction reaction is 16-20 h, and the temperature for the reduction reaction is 20-40 °C. Under the reaction temperature and time conditions, the reaction efficiency is high, and energy consumption is relatively less. Any PH condition conductive to the synthesis of the hydrazine compound is applicable to the present invention. From the perspective of conditions that suitable for the reduction reaction of reactants, in a preferred embodiment, the pH value for the reduction reaction is 7.5-10.

The present invention is further described in detail below with reference to specific embodiments, and the embodiments cannot be construed as limiting the scope of protection claimed in the present invention.

It is noted that the method below for detecting a conversion rate of the product hydrazine compound includes GC detection and HPLC detection, and data obtained can indicate the conversion rate for the enzyme-catalyzed reaction used in the preparation method of the present invention. The selection of the detection method is based on the state of the product; if the product has a low boiling point (≤200 °C), GC detection is used; and if the product has a high boiling point (greater than 200 °C), HPLC detection is used.

The following embodiments that label a chiral configuration of the product are embodiments that may generate a single chiral product. Hydrazine compounds with single chirality are a very important class of structural units found in pharmaceuticals, agrochemicals, natural products, and chiral catalysts, and have widespread applications and significant importance.

### Embodiment 1:

In a 10 mL reaction bottle, 306 imine reductase enzyme solutions (a mass ratio of acetone to imine reductase in the enzyme solution was 1:8) shown in Table 1 were respectively added, then 11.5 mg of GDH, 21.6 mg of Glu, 1.33 mg of NAD⁺, 1.53 mg of NADP⁺, 11.5 mg (100 mM) of acetone 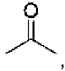 and 12 mg (120 mM) of hydrazine hydrate were added. The total volume was adjusted to 2 mL with100 mM Tris-HCl buffer (pH 8.5), and the reaction was performed for 20 h at 30 °C through shaking. 500 µL of the system was taken, the reaction was terminated with 100 µL of a 0.1 M sodium hydroxide solution, centrifugation was performed for 10 min at 12000 rpm, extraction was performed using ethyl acetate, and then GC or LC-MS detection was performed to determine a conversion rate, as shown in Table 2 below.

**Table 2:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | + | 233 | + |
| 3 | + | 80 | + | 157 | + | 234 | + |
| 4 | ++ | 81 | + | 158 | + | 235 | + |
| 5 | + | 82 | + | 159 | + | 236 | + |
| 6 | + | 83 | + | 160 | + | 237 | + |
| 7 | + | 84 | + | 161 | + | 238 | + |
| 8 | + | 85 | + | 162 | + | 239 | + |
| 9 | +++ | 86 | + | 163 | + | 240 | + |
| 10 | + | 87 | + | 164 | +++ | 241 | ++ |
| 11 | + | 88 | +++ | 165 | + | 242 | + |
| 12 | + | 89 | + | 166 | + | 243 | + |
| 13 | + | 90 | + | 167 | + | 244 | + |
| 14 | + | 91 | + | 168 | + | 245 | + |
| 15 | +++ | 92 | + | 169 | + | 246 | + |
| 16 | + | 93 | + | 170 | + | 247 | + |
| 17 | + | 94 | + | 171 | + | 248 | + |
| 18 | +++ | 95 | + | 172 | + | 249 | + |
| 19 | + | 96 | + | 173 | + | 250 | + |
| 20 | +++ | 97 | + | 174 | + | 251 | + |
| 21 | + | 98 | + | 175 | + | 252 | +++ |
| 22 | ++++ | 99 | + | 176 | + | 253 | + |
| 23 | + | 100 | + | 177 | + | 254 | + |
| 24 | + | 101 | + | 178 | + | 255 | + |
| 25 | + | 102 | + | 179 | + | 256 | + |
| 26 | + | 103 | + | 180 | + | 257 | + |
| 27 | +++ | 104 | + | 181 | + | 258 | ++ |
| 28 | + | 105 | +++ | 182 | ++ | 259 | + |
| 29 | + | 106 | + | 183 | + | 260 | + |
| 30 | + | 107 | + | 184 | + | 261 | + |
| 31 | +++ | 108 | + | 185 | + | 262 | + |
| 32 | + | 109 | + | 186 | + | 263 | + |
| 33 | + | 110 | + | 187 | + | 264 | + |
| 34 | + | 111 | + | 188 | + | 265 | + |
| 35 | + | 112 | +++ | 189 | + | 266 | + |
| 36 | + | 113 | + | 190 | + | 267 | + |
| 37 | ++ | 114 | + | 191 | + | 268 | + |
| 38 | + | 115 | + | 192 | + | 269 | + |
| 39 | + | 116 | + | 193 | +++ | 270 | ++ |
| 40 | + | 117 | + | 194 | + | 271 | + |
| 41 | + | 118 | + | 195 | + | 272 | + |
| 42 | + | 119 | + | 196 | + | 273 | + |
| 43 | + | 120 | + | 197 | + | 274 | + |
| 44 | + | 121 | + | 198 | + | 275 | + |
| 45 | + | 122 | + | 199 | + | 276 | + |
| 46 | + | 123 | + | 200 | + | 277 | + |
| 47 | + | 124 | + | 201 | + | 278 | + |
| 48 | + | 125 | + | 202 | + | 279 | +++ |
| 49 | + | 126 | +++ | 203 | + | 280 | + |
| 50 | + | 127 | + | 204 | + | 281 | + |
| 51 | +++ | 128 | + | 205 | + | 282 | + |
| 52 | + | 129 | + | 206 | ++ | 283 | + |
| 53 | + | 130 | + | 207 | + | 284 | + |
| 54 | + | 131 | + | 208 | + | 285 | + |
| 55 | + | 132 | + | 209 | ++ | 286 | ++ |
| 56 | + | 133 | + | 210 | + | 287 | + |
| 57 | + | 134 | +++ | 211 | + | 288 | ++ |
| 58 | + | 135 | + | 212 | + | 289 | + |
| 59 | + | 136 | + | 213 | + | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | + | 140 | + | 217 | + | 294 | + |
| 64 | + | 141 | + | 218 | + | 295 | + |
| 65 | + | 142 | + | 219 | + | 296 | + |
| 66 | +++ | 143 | + | 220 | +++ | 297 | ++ |
| 67 | + | 144 | + | 221 | + | 298 | + |
| 68 | + | 145 | + | 222 | + | 299 | + |
| 69 | + | 146 | + | 223 | + | 300 | + |
| 70 | + | 147 | + | 224 | + | 301 | +++ |
| 71 | + | 148 | + | 225 | + | 302 | + |
| 72 | + | 149 | +++ | 226 | + | 303 | + |
| 73 | + | 150 | + | 227 | + | 304 | + |
| 74 | + | 151 | + | 228 | ++ | 305 | + |
| 75 | + | 152 | + | 229 | + | 306 | + |
| 76 | ++ | 153 | + | 230 | + | | |
| 77 | + | 154 | ++ | 231 | + | | |

In the table above, + indicated that the conversion rate was 0.01-1%, ++ indicated that the conversion rate was 1%-10%, +++ indicated that the conversion rate was 10%-30%, ++++ indicated that the conversion rate was 30-50%, and +++++ indicated that the conversion rate was 50% or higher.

### Embodiment 2:

In a 10 mL reaction bottle, 306 imine reductase enzyme solutions (a mass ratio of substrate ketone to imine reductase in the enzyme solution was 1:10) shown in Table 1 were respectively added, then 5.48 mg of GDH, 21.6 mg of Glu, 1.33 mg of NAD⁺, 1.53 mg of NADP⁺, 5.48 mg (20 mM) of 3-cyclopentyl-3-oxo-propionitrile 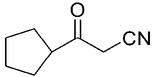 (dissolved in 10 µL of DMSO), and 20 mg (200 mM) of hydrazine hydrate were added. The total volume was adjusted to 100 mM Tris-HCl buffer (pH 9.0), and the reaction was performed for 16 h at 25 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 3 below. Chirality detection was performed by selecting part of the enzymes, for example, for enzymes 4, 37, 72, and the like, the obtained products were predominantly in an R configuration, and for enzymes 5, 18, 24, 17, 25, and the like, the obtained products were predominantly in an S configuration.

**Table 3:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | + | 233 | + |
| 3 | ++ | 80 | + | 157 | ++ | 234 | + |
| 4 | ++ | 81 | + | 158 | + | 235 | + |
| 5 | +++ | 82 | + | 159 | + | 236 | + |
| 6 | + | 83 | ++ | 160 | + | 237 | + |
| 7 | + | 84 | + | 161 | + | 238 | + |
| 8 | + | 85 | + | 162 | + | 239 | +++ |
| 9 | + | 86 | + | 163 | + | 240 | + |
| 10 | + | 87 | + | 164 | + | 241 | + |
| 11 | + | 88 | + | 165 | + | 242 | + |
| 12 | + | 89 | + | 166 | + | 243 | + |
| 13 | + | 90 | + | 167 | + | 244 | + |
| 14 | + | 91 | + | 168 | + | 245 | + |
| 15 | + | 92 | + | 169 | + | 246 | + |
| 16 | + | 93 | + | 170 | + | 247 | + |
| 17 | +++ | 94 | + | 171 | + | 248 | + |
| 18 | ++++ | 95 | + | 172 | + | 249 | + |
| 19 | + | 96 | + | 173 | + | 250 | + |
| 20 | + | 97 | + | 174 | +++ | 251 | + |
| 21 | + | 98 | + | 175 | + | 252 | + |
| 22 | ++ | 99 | + | 176 | + | 253 | + |
| 23 | + | 100 | + | 177 | + | 254 | ++ |
| 24 | ++++ | 101 | + | 178 | + | 255 | + |
| 25 | ++ | 102 | + | 179 | + | 256 | + |
| 26 | ++ | 103 | + | 180 | + | 257 | + |
| 27 | + | 104 | + | 181 | + | 258 | + |
| 28 | ++ | 105 | + | 182 | + | 259 | + |
| 29 | + | 106 | + | 183 | + | 260 | + |
| 30 | + | 107 | + | 184 | + | 261 | + |
| 31 | ++ | 108 | + | 185 | + | 262 | + |
| 32 | + | 109 | ++ | 186 | + | 263 | + |
| 33 | ++ | 110 | + | 187 | ++ | 264 | + |
| 34 | + | 111 | + | 188 | + | 265 | + |
| 35 | + | 112 | + | 189 | + | 266 | + |
| 36 | + | 113 | + | 190 | + | 267 | + |
| 37 | +++ | 114 | + | 191 | + | 268 | + |
| 38 | + | 115 | + | 192 | + | 269 | + |
| 39 | + | 116 | + | 193 | + | 270 | + |
| 40 | + | 117 | + | 194 | + | 271 | + |
| 41 | ++ | 118 | + | 195 | + | 272 | + |
| 42 | + | 119 | + | 196 | + | 273 | ++ |
| 43 | + | 120 | + | 197 | + | 274 | + |
| 44 | + | 121 | + | 198 | + | 275 | + |
| 45 | + | 122 | ++ | 199 | + | 276 | + |
| 46 | + | 123 | + | 200 | ++ | 277 | + |
| 47 | + | 124 | + | 201 | + | 278 | + |
| 48 | + | 125 | + | 202 | + | 279 | + |
| 49 | + | 126 | + | 203 | + | 280 | + |
| 50 | + | 127 | + | 204 | + | 281 | + |
| 51 | + | 128 | + | 205 | + | 282 | + |
| 52 | + | 129 | + | 206 | + | 283 | +++ |
| 53 | + | 130 | + | 207 | + | 284 | + |
| 54 | + | 131 | + | 208 | + | 285 | + |
| 55 | + | 132 | + | 209 | + | 286 | + |
| 56 | + | 133 | + | 210 | + | 287 | + |
| 57 | + | 134 | ++ | 211 | + | 288 | + |
| 58 | + | 135 | + | 212 | + | 289 | + |
| 59 | ++ | 136 | + | 213 | ++ | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | + | 140 | + | 217 | + | 294 | + |
| 64 | + | 141 | + | 218 | + | 295 | + |
| 65 | + | 142 | + | 219 | + | 296 | + |
| 66 | + | 143 | + | 220 | + | 297 | + |
| 67 | + | 144 | + | 221 | + | 298 | ++ |
| 68 | + | 145 | + | 222 | + | 299 | + |
| 69 | + | 146 | + | 223 | + | 300 | + |
| 70 | + | 147 | + | 224 | + | 301 | + |
| 71 | + | 148 | + | 225 | + | 302 | + |
| 72 | +++ | 149 | + | 226 | + | 303 | + |
| 73 | + | 150 | + | 227 | + | 304 | + |
| 74 | + | 151 | + | 228 | + | 305 | + |
| 75 | + | 152 | + | 229 | + | 306 | + |
| 76 | + | 153 | + | 230 | ++ | | |
| 77 | + | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 3:

In a 10 mL reaction bottle, 306 imine reductase enzyme solutions (a mass ratio of substrate ketone to imine reductase in the enzyme solution was 1:6) shown in Table 1 were respectively added, then 15.68 mg of GDH, 21.6 mg of Glu, 1.33 mg of NAD⁺, 1.53 mg of NADP⁺, 15.68 mg (80 mM) of cyclohexanone 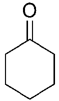 (dissolved in 10 µL of DMSO), and 9.6 mg (96 mM) of hydrazine hydrate were added. The total volume was adjusted to 2 mL with 100 mM Tris-HCl buffer (pH 8.0), and the reaction was performed for 16 h at 20 °C through shaking. 500 µL of the system was taken, the reaction was terminated with 100 µL of a 0.1 M sodium hydroxide solution, centrifugation was performed for 10 min at 12000 rpm, extraction was performed using ethyl acetate, and then GC or LC-MS detection was performed to determine a conversion rate, as shown in Table 4 below.

**Table 4:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | +++ | 155 | + | 232 | ++ |
| 2 | +++ | 79 | ++++ | 156 | ++++ | 233 | + |
| 3 | ++++ | 80 | ++++ | 157 | + | 234 | +++ |
| 4 | ++++ | 81 | +++ | 158 | ++++ | 235 | ++++ |
| 5 | ++++ | 82 | + | 159 | + | 236 | ++++ |
| 6 | + | 83 | + | 160 | + | 237 | + |
| 7 | ++++ | 84 | +++ | 161 | ++++ | 238 | + |
| 8 | +++ | 85 | ++++ | 162 | ++++ | 239 | +++ |
| 9 | +++ | 86 | ++++ | 163 | + | 240 | ++++ |
| 10 | ++++ | 87 | ++++ | 164 | ++++ | 241 | + |
| 11 | + | 88 | +++ | 165 | + | 242 | ++++ |
| 12 | ++++ | 89 | ++++ | 166 | +++ | 243 | + |
| 13 | ++++ | 90 | +++ | 167 | +++ | 244 | ++++ |
| 14 | ++++ | 91 | +++ | 168 | ++++ | 245 | ++ |
| 15 | +++ | 92 | +++ | 169 | + | 246 | + |
| 16 | +++++ | 93 | ++++ | 170 | +++ | 247 | + |
| 17 | ++++ | 94 | ++++ | 171 | + | 248 | +++ |
| 18 | ++++ | 95 | ++ | 172 | ++++ | 249 | + |
| 19 | ++++ | 96 | + | 173 | ++++ | 250 | + |
| 20 | +++++ | 97 | +++ | 174 | + | 251 | ++++ |
| 21 | ++++ | 98 | + | 175 | ++++ | 252 | + |
| 22 | ++++ | 99 | + | 176 | + | 253 | +++ |
| 23 | +++++ | 100 | ++++ | 177 | ++++ | 254 | + |
| 24 | +++++ | 101 | ++++ | 178 | + | 255 | ++++ |
| 25 | ++++ | 102 | +++ | 179 | ++++ | 256 | ++++ |
| 26 | ++++ | 103 | ++++ | 180 | ++++ | 257 | + |
| 27 | ++++ | 104 | ++++ | 181 | +++ | 258 | + |
| 28 | ++++ | 105 | + | 182 | + | 259 | +++ |
| 29 | ++++ | 106 | + | 183 | +++ | 260 | + |
| 30 | + | 107 | ++++ | 184 | ++++ | 261 | + |
| 31 | ++++ | 108 | ++++ | 185 | + | 262 | + |
| 32 | + | 109 | + | 186 | ++ | 263 | ++++ |
| 33 | ++++ | 110 | ++++ | 187 | ++++ | 264 | + |
| 34 | +++ | 111 | + | 188 | + | 265 | ++++ |
| 35 | + | 112 | ++++ | 189 | ++++ | 266 | + |
| 36 | ++++ | 113 | ++ | 190 | ++++ | 267 | + |
| 37 | ++++ | 114 | ++++ | 191 | +++ | 268 | +++ |
| 38 | + | 115 | ++++ | 192 | + | 269 | + |
| 39 | + | 116 | +++ | 193 | ++++ | 270 | + |
| 40 | ++++ | 117 | + | 194 | ++++ | 271 | + |
| 41 | ++++ | 118 | ++++ | 195 | +++ | 272 | ++++ |
| 42 | ++++ | 119 | ++++ | 196 | + | 273 | + |
| 43 | ++++ | 120 | + | 197 | ++++ | 274 | +++++ |
| 44 | +++ | 121 | +++ | 198 | +++ | 275 | ++ |
| 45 | + | 122 | +++ | 199 | + | 276 | + |
| 46 | + | 123 | ++++ | 200 | ++++ | 277 | +++ |
| 47 | +++++ | 124 | + | 201 | + | 278 | + |
| 48 | + | 125 | ++++ | 202 | +++ | 279 | ++++ |
| 49 | + | 126 | ++++ | 203 | + | 280 | + |
| 50 | + | 127 | +++++ | 204 | ++++ | 281 | ++++ |
| 51 | + | 128 | + | 205 | +++ | 282 | ++++ |
| 52 | + | 129 | ++++ | 206 | ++++ | 283 | ++++ |
| 53 | + | 130 | ++++ | 207 | + | 284 | +++++ |
| 54 | + | 131 | + | 208 | ++++ | 285 | + |
| 55 | + | 132 | ++ | 209 | + | 286 | +++ |
| 56 | + | 133 | ++++ | 210 | + | 287 | ++++ |
| 57 | + | 134 | +++ | 211 | +++ | 288 | +++ |
| 58 | + | 135 | ++++ | 212 | +++ | 289 | +++++ |
| 59 | +++++ | 136 | +++ | 213 | ++++ | 290 | ++++ |
| 60 | +++ | 137 | + | 214 | ++ | 291 | ++++ |
| 61 | ++++ | 138 | ++++ | 215 | + | 292 | +++++ |
| 62 | + | 139 | ++++ | 216 | ++++ | 293 | ++ |
| 63 | ++++ | 140 | +++ | 217 | + | 294 | ++++ |
| 64 | ++++ | 141 | + | 218 | ++++ | 295 | +++ |
| 65 | + | 142 | ++++ | 219 | ++++ | 296 | + |
| 66 | + | 143 | + | 220 | + | 297 | ++++ |
| 67 | ++++ | 144 | ++++ | 221 | +++ | 298 | +++ |
| 68 | + | 145 | + | 222 | + | 299 | + |
| 69 | +++ | 146 | + | 223 | + | 300 | ++++ |
| 70 | ++++ | 147 | + | 224 | +++ | 301 | ++++ |
| 71 | ++++ | 148 | ++++ | 225 | ++++ | 302 | + |
| 72 | +++++ | 149 | + | 226 | + | 303 | ++ |
| 73 | ++++ | 150 | + | 227 | +++ | 304 | + |
| 74 | + | 151 | ++++ | 228 | + | 305 | +++ |
| 75 | ++++ | 152 | ++++ | 229 | ++++ | 306 | ++++ |
| 76 | ++++ | 153 | ++ | 230 | ++++ | | |
| 77 | ++++ | 154 | +++ | 231 | ++++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 4:

In a 10 mL reaction bottle, 306 imine reductase enzyme solutions (a mass ratio of substrate ketone to imine reductase in the enzyme solution was 1:10) shown in Table 1 were respectively added, then 14.6 mg of GDH, 21.6 mg of Glu, 1.33 mg of NAD⁺, 1.53 mg of NADP⁺, 14.6 mg (50 mM) of beta-tetralone 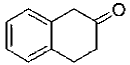 (dissolved in 10 µL of DMSO), and 5 mg (50 mM) of hydrazine hydrate were added. The total volume was adjusted to 2 mL with 100 mM Tris-HCl buffer (pH 7.5), and the reaction was performed for 18 h at 35 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 5 below.

**Table 5:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | +++ | 233 | + |
| 3 | + | 80 | ++ | 157 | + | 234 | ++++ |
| 4 | + | 81 | + | 158 | ++++ | 235 | + |
| 5 | ++ | 82 | + | 159 | + | 236 | + |
| 6 | + | 83 | + | 160 | + | 237 | + |
| 7 | + | 84 | + | 161 | + | 238 | + |
| 8 | ++ | 85 | + | 162 | + | 239 | + |
| 9 | + | 86 | ++++ | 163 | ++ | 240 | + |
| 10 | + | 87 | + | 164 | + | 241 | + |
| 11 | + | 88 | + | 165 | + | 242 | + |
| 12 | ++++ | 89 | + | 166 | + | 243 | + |
| 13 | + | 90 | + | 167 | ++ | 244 | +++ |
| 14 | ++++ | 91 | + | 168 | + | 245 | + |
| 15 | + | 92 | + | 169 | + | 246 | + |
| 16 | ++++ | 93 | + | 170 | + | 247 | + |
| 17 | ++++ | 94 | + | 171 | + | 248 | + |
| 18 | + | 95 | + | 172 | + | 249 | + |
| 19 | + | 96 | + | 173 | + | 250 | ++ |
| 20 | ++++ | 97 | + | 174 | + | 251 | + |
| 21 | + | 98 | + | 175 | +++ | 252 | + |
| 22 | + | 99 | + | 176 | + | 253 | + |
| 23 | ++++ | 100 | + | 177 | + | 254 | + |
| 24 | + | 101 | + | 178 | + | 255 | + |
| 25 | ++ | 102 | + | 179 | + | 256 | + |
| 26 | + | 103 | + | 180 | + | 257 | + |
| 27 | + | 104 | + | 181 | + | 258 | + |
| 28 | + | 105 | + | 182 | + | 259 | + |
| 29 | + | 106 | ++++ | 183 | + | 260 | ++++ |
| 30 | + | 107 | ++++ | 184 | +++ | 261 | + |
| 31 | + | 108 | + | 185 | + | 262 | + |
| 32 | + | 109 | + | 186 | + | 263 | + |
| 33 | + | 110 | ++++ | 187 | + | 264 | + |
| 34 | ++++ | 111 | + | 188 | + | 265 | ++ |
| 35 | | 112 | + | 189 | + | 266 | + |
| 36 | +++ | 113 | + | 190 | + | 267 | + |
| 37 | + | 114 | + | 191 | ++ | 268 | + |
| 38 | + | 115 | + | 192 | + | 269 | + |
| 39 | + | 116 | + | 193 | + | 270 | + |
| 40 | ++++ | 117 | + | 194 | + | 271 | + |
| 41 | + | 118 | + | 195 | + | 272 | + |
| 42 | ++++ | 119 | + | 196 | + | 273 | + |
| 43 | ++++ | 120 | + | 197 | + | 274 | + |
| 44 | + | 121 | + | 198 | ++++ | 275 | + |
| 45 | + | 122 | ++++ | 199 | + | 276 | + |
| 46 | + | 123 | + | 200 | + | 277 | ++ |
| 47 | ++++ | 124 | + | 201 | + | 278 | + |
| 48 | + | 125 | + | 202 | + | 279 | + |
| 49 | + | 126 | + | 203 | ++ | 280 | + |
| 50 | + | 127 | + | 204 | + | 281 | + |
| 51 | + | 128 | ++++ | 205 | + | 282 | + |
| 52 | ++ | 129 | + | 206 | + | 283 | + |
| 53 | + | 130 | + | 207 | + | 284 | + |
| 54 | + | 131 | + | 208 | + | 285 | + |
| 55 | + | 132 | + | 209 | + | 286 | + |
| 56 | + | 133 | + | 210 | + | 287 | + |
| 57 | + | 134 | + | 211 | + | 288 | + |
| 58 | ++ | 135 | ++ | 212 | + | 289 | +++ |
| 59 | + | 136 | + | 213 | ++++ | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | + | 140 | ++++ | 217 | ++ | 294 | + |
| 64 | + | 141 | + | 218 | + | 295 | ++ |
| 65 | + | 142 | + | 219 | + | 296 | + |
| 66 | ++++ | 143 | + | 220 | +++ | 297 | + |
| 67 | + | 144 | +++ | 221 | + | 298 | + |
| 68 | + | 145 | + | 222 | + | 299 | ++++ |
| 69 | + | 146 | + | 223 | + | 300 | + |
| 70 | + | 147 | + | 224 | + | 301 | + |
| 71 | + | 148 | + | 225 | + | 302 | + |
| 72 | + | 149 | + | 226 | + | 303 | +++ |
| 73 | + | 150 | + | 227 | + | 304 | + |
| 74 | ++ | 151 | + | 228 | + | 305 | ++ |
| 75 | + | 152 | + | 229 | + | 306 | + |
| 76 | + | 153 | + | 230 | + | | |
| 77 | + | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 5:

In a 10 mL reaction bottle, 306 imine reductase enzyme solutions (a mass ratio of substrate ketone to imine reductase in the enzyme solution was 1:10) shown in Table 1 were respectively added, then 3.64 mg of GDH, 21.6 mg of Glu, 1.33 mg of NAD⁺, 1.53 mg of NADP⁺, 3.64 mg (10 mM) of 6,8-difluoro-2-tetralone 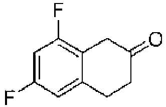 (dissolved in 10 µL of DMSO), and 10 mg (100 mM) of hydrazine hydrate were added. The total volume was adjusted to 2 mL with 100 mM Tris-HCl buffer (pH 7.5), and the reaction was performed for 16 h at 30 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 6 below.

**Table 6:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | ++ | 232 | + |
| 2 | + | 79 | ++++ | 156 | ++ | 233 | + |
| 3 | + | 80 | + | 157 | + | 234 | + |
| 4 | + | 81 | + | 158 | + | 235 | ++++ |
| 5 | + | 82 | + | 159 | + | 236 | + |
| 6 | + | 83 | + | 160 | +++ | 237 | + |
| 7 | + | 84 | + | 161 | + | 238 | ++++ |
| 8 | + | 85 | ++++ | 162 | + | 239 | + |
| 9 | + | 86 | ++++ | 163 | ++ | 240 | + |
| 10 | + | 87 | ++++ | 164 | ++ | 241 | + |
| 11 | + | 88 | + | 165 | + | 242 | +++ |
| 12 | +++ | 89 | + | 166 | + | 243 | + |
| 13 | + | 90 | ++++ | 167 | + | 244 | ++ |
| 14 | ++++ | 91 | + | 168 | ++++ | 245 | ++ |
| 15 | + | 92 | + | 169 | + | 246 | + |
| 16 | +++++ | 93 | + | 170 | + | 247 | ++++ |
| 17 | ++++ | 94 | + | 171 | ++ | 248 | + |
| 18 | +++ | 95 | ++ | 172 | ++ | 249 | + |
| 19 | +++ | 96 | + | 173 | ++ | 250 | ++++ |
| 20 | ++++ | 97 | + | 174 | + | 251 | + |
| 21 | +++ | 98 | + | 175 | + | 252 | +++ |
| 22 | + | 99 | + | 176 | +++ | 253 | + |
| 23 | ++++ | 100 | + | 177 | ++ | 254 | + |
| 24 | + | 101 | + | 178 | + | 255 | ++ |
| 25 | + | 102 | + | 179 | +++ | 256 | ++ |
| 26 | + | 103 | + | 180 | ++ | 257 | + |
| 27 | + | 104 | +++ | 181 | + | 258 | + |
| 28 | + | 105 | ++++ | 182 | + | 259 | + |
| 29 | + | 106 | + | 183 | ++++ | 260 | ++++ |
| 30 | + | 107 | +++ | 184 | + | 261 | + |
| 31 | + | 108 | ++++ | 185 | ++ | 262 | ++++ |
| 32 | + | 109 | + | 186 | ++ | 263 | + |
| 33 | + | 110 | + | 187 | + | 264 | ++ |
| 34 | +++ | 111 | ++ | 188 | ++++ | 265 | + |
| 35 | + | 112 | + | 189 | + | 266 | ++++ |
| 36 | +++ | 113 | + | 190 | ++ | 267 | ++ |
| 37 | +++ | 114 | + | 191 | ++ | 268 | + |
| 38 | +++ | 115 | ++++ | 192 | + | 269 | + |
| 39 | ++++ | 116 | + | 193 | + | 270 | +++ |
| 40 | +++ | 117 | + | 194 | +++ | 271 | ++ |
| 41 | + | 118 | ++ | 195 | + | 272 | + |
| 42 | +++ | 119 | ++ | 196 | + | 273 | + |
| 43 | +++++ | 120 | + | 197 | + | 274 | + |
| 44 | + | 121 | + | 198 | +++ | 275 | ++++ |
| 45 | + | 122 | + | 199 | + | 276 | + |
| 46 | + | 123 | ++++ | 200 | + | 277 | ++ |
| 47 | ++++ | 124 | ++ | 201 | ++++ | 278 | ++++ |
| 48 | + | 125 | + | 202 | + | 279 | + |
| 49 | + | 126 | + | 203 | ++++ | 280 | + |
| 50 | + | 127 | ++ | 204 | + | 281 | + |
| 51 | + | 128 | ++ | 205 | ++ | 282 | +++ |
| 52 | + | 129 | + | 206 | ++ | 283 | + |
| 53 | + | 130 | + | 207 | + | 284 | ++ |
| 54 | + | 131 | +++ | 208 | + | 285 | ++ |
| 55 | + | 132 | ++++ | 209 | ++++ | 286 | + |
| 56 | + | 133 | + | 210 | ++++ | 287 | + |
| 57 | + | 134 | + | 211 | + | 288 | ++++ |
| 58 | + | 135 | + | 212 | + | 289 | + |
| 59 | + | 136 | ++ | 213 | + | 290 | ++++ |
| 60 | + | 137 | ++ | 214 | + | 291 | + |
| 61 | + | 138 | + | 215 | ++++ | 292 | ++ |
| 62 | + | 139 | +++ | 216 | + | 293 | + |
| 63 | + | 140 | + | 217 | +++ | 294 | +++ |
| 64 | + | 141 | ++++ | 218 | + | 295 | + |
| 65 | + | 142 | + | 219 | + | 296 | ++ |
| 66 | + | 143 | ++++ | 220 | ++ | 297 | + |
| 67 | +++ | 144 | ++ | 221 | ++ | 298 | + |
| 68 | + | 145 | ++ | 222 | + | 299 | + |
| 69 | + | 146 | + | 223 | + | 300 | +++ |
| 70 | + | 147 | + | 224 | + | 301 | ++ |
| 71 | +++ | 148 | ++++ | 225 | +++ | 302 | + |
| 72 | + | 149 | ++ | 226 | + | 303 | ++++ |
| 73 | +++ | 150 | + | 227 | + | 304 | + |
| 74 | + | 151 | +++ | 228 | ++++ | 305 | ++++ |
| 75 | + | 152 | + | 229 | + | 306 | ++ |
| 76 | + | 153 | + | 230 | ++ | | |
| 77 | ++++ | 154 | ++ | 231 | ++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 6:

In a 10 mL reaction bottle, 306 imine reductase enzyme solutions (a mass ratio of substrate ketone to imine reductase in the enzyme solution was 1:8) shown in Table 1 were respectively added, then 8.12 mg of GDH, 21.6 mg of Glu, 1.33 mg of NAD⁺, 1.53 mg of NADP⁺, 8.12 mg (20 mM) of 1-benzyl-3-methyl-4-oxopiperidine 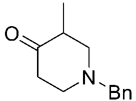 (dissolved in 10 _{IJ}L of DMSO), and 10 mg (100 mM) of hydrazine hydrate were added. The total volume was adjusted to 2 mL with 100 mM Tris-HCI buffer (pH 10.0), and the reaction was performed for 16 h at 40 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 7 below.

**Table 7:**

| Enzyme number | Conversion rate | Enzyme number | Conversion rate | Enzyme number | Conversion rate | Enzyme number | Conversion rate |
|---|---|---|---|---|---|---|---|
| 1 | +++ | 78 | + | 155 | + | 232 | ++++ |
| 2 | + | 79 | +++ | 156 | ++++ | 233 | ++++ |
| 3 | +++ | 80 | + | 157 | + | 234 | + |
| 4 | + | 81 | + | 158 | ++ | 235 | + |
| 5 | +++++ | 82 | + | 159 | ++ | 236 | + |
| 6 | ++ | 83 | + | 160 | + | 237 | + |
| 7 | + | 84 | +++ | 161 | + | 238 | +++++ |
| 8 | + | 85 | +++++ | 162 | ++++ | 239 | + |
| 9 | + | 86 | +++ | 163 | ++ | 240 | + |
| 10 | + | 87 | ++++ | 164 | + | 241 | + |
| 11 | + | 88 | + | 165 | + | 242 | ++++ |
| 12 | ++ | 89 | + | 166 | +++++ | 243 | + |
| 13 | + | 90 | +++ | 167 | ++ | 244 | ++++ |
| 14 | ++++ | 91 | +++ | 168 | ++ | 245 | + |
| 15 | + | 92 | + | 169 | + | 246 | + |
| 16 | +++++ | 93 | +++ | 170 | + | 247 | + |
| 17 | ++++ | 94 | + | 171 | + | 248 | + |
| 18 | + | 95 | + | 172 | ++++ | 249 | + |
| 19 | ++++ | 96 | + | 173 | ++ | 250 | ++++ |
| 20 | ++++ | 97 | + | 174 | ++++ | 251 | + |
| 21 | +++++ | 98 | + | 175 | + | 252 | + |
| 22 | +++ | 99 | + | 176 | + | 253 | +++++ |
| 23 | ++++ | 100 | + | 177 | ++ | 254 | + |
| 24 | +++++ | 101 | + | 178 | + | 255 | + |
| 25 | +++ | 102 | + | 179 | ++ | 256 | ++++ |
| 26 | + | 103 | + | 180 | + | 257 | + |
| 27 | +++++ | 104 | +++++ | 181 | + | 258 | + |
| 28 | + | 105 | ++ | 182 | + | 259 | + |
| 29 | + | 106 | ++++ | 183 | ++++ | 260 | + |
| 30 | + | 107 | +++++ | 184 | + | 261 | ++ |
| 31 | + | 108 | ++ | 185 | + | 262 | + |
| 32 | + | 109 | + | 186 | + | 263 | + |
| 33 | + | 110 | + | 187 | +++++ | 264 | + |
| 34 | +++ | 111 | ++ | 188 | + | 265 | ++++ |
| 35 | + | 112 | ++ | 189 | ++ | 266 | + |
| 36 | + | 113 | +++++ | 190 | + | 267 | + |
| 37 | +++++ | 114 | ++ | 191 | + | 268 | + |
| 38 | ++++ | 115 | ++++ | 192 | ++++ | 269 | + |
| 39 | + | 116 | + | 193 | ++ | 270 | + |
| 40 | +++++ | 117 | ++++ | 194 | ++++ | 271 | +++++ |
| 41 | + | 118 | ++ | 195 | ++++ | 272 | + |
| 42 | + | 119 | +++++ | 196 | + | 273 | + |
| 43 | ++++ | 120 | ++ | 197 | + | 274 | + |
| 44 | + | 121 | ++++ | 198 | + | 275 | + |
| 45 | + | 122 | ++++ | 199 | ++++ | 276 | + |
| 46 | + | 123 | + | 200 | + | 277 | ++++ |
| 47 | +++ | 124 | ++++ | 201 | + | 278 | + |
| 48 | + | 125 | + | 202 | ++++ | 279 | ++++ |
| 49 | + | 126 | + | 203 | ++++ | 280 | + |
| 50 | + | 127 | + | 204 | ++ | 281 | + |
| 51 | + | 128 | ++ | 205 | + | 282 | ++ |
| 52 | + | 129 | + | 206 | ++ | 283 | ++ |
| 53 | + | 130 | + | 207 | ++ | 284 | + |
| 54 | + | 131 | +++++ | 208 | + | 285 | + |
| 55 | + | 132 | + | 209 | + | 286 | + |
| 56 | + | 133 | + | 210 | + | 287 | + |
| 57 | + | 134 | ++++ | 211 | +++++ | 288 | + |
| 58 | + | 135 | + | 212 | ++ | 289 | +++++ |
| 59 | + | 136 | +++++ | 213 | + | 290 | + |
| 60 | + | 137 | + | 214 | ++++ | 291 | + |
| 61 | +++++ | 138 | ++ | 215 | ++ | 292 | + |
| 62 | + | 139 | + | 216 | ++++ | 293 | ++++ |
| 63 | +++ | 140 | ++ | 217 | + | 294 | ++++ |
| 64 | + | 141 | ++++ | 218 | + | 295 | + |
| 65 | + | 142 | + | 219 | + | 296 | ++++ |
| 66 | + | 143 | + | 220 | +++++ | 297 | + |
| 67 | + | 144 | ++ | 221 | + | 298 | + |
| 68 | + | 145 | ++ | 222 | ++++ | 299 | + |
| 69 | + | 146 | +++++ | 223 | + | 300 | ++++ |
| 70 | + | 147 | + | 224 | + | 301 | + |
| 71 | + | 148 | + | 225 | + | 302 | + |
| 72 | + | 149 | + | 226 | ++++ | 303 | +++++ |
| 73 | + | 150 | ++ | 227 | ++++ | 304 | ++++ |
| 74 | + | 151 | ++ | 228 | ++++ | 305 | ++ |
| 75 | + | 152 | + | 229 | + | 306 | ++ |
| 76 | + | 153 | +++++ | 230 | + | | |
| 77 | + | 154 | ++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 7:

In a 10 mL reaction bottle, 306 imine reductase enzyme solutions (a mass ratio of substrate ketone to imine reductase in the enzyme solution was 1:5) shown in Table 1 were respectively added, then 5.12 mg of GDH, 21.6 mg of Glu, 1.33 mg of NAD⁺, 1.53 mg of NADP⁺, 5.12 mg (20 mM) of 1-(tetrahydro-2H-pyran-4-yl)ethanone 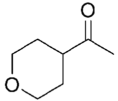 (dissolved in 10 µL of DMSO), and 10 mg (100 mM) of hydrazine hydrate were added. The total volume was adjusted to 2 mL with 100 mM Tris-HCI buffer (pH 9.0), and the reaction was performed for 16 h at 30 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 8 below.

**Table 8:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | +++ | 155 | + | 232 | ++ |
| 2 | +++ | 79 | ++++ | 156 | ++++ | 233 | + |
| 3 | +++ | 80 | ++++ | 157 | + | 234 | +++ |
| 4 | ++++ | 81 | +++ | 158 | ++++ | 235 | ++++ |
| 5 | +++++ | 82 | + | 159 | + | 236 | ++++ |
| 6 | + | 83 | + | 160 | + | 237 | + |
| 7 | ++++ | 84 | +++ | 161 | ++++ | 238 | + |
| 8 | +++ | 85 | ++++ | 162 | ++++ | 239 | +++ |
| 9 | +++ | 86 | ++++ | 163 | + | 240 | ++++ |
| 10 | ++++ | 87 | ++++ | 164 | ++++ | 241 | + |
| 11 | + | 88 | +++ | 165 | + | 242 | ++++ |
| 12 | ++++ | 89 | ++++ | 166 | +++ | 243 | + |
| 13 | ++++ | 90 | +++ | 167 | +++ | 244 | ++++ |
| 14 | ++++ | 91 | +++ | 168 | ++++ | 245 | ++ |
| 15 | +++ | 92 | +++ | 169 | + | 246 | + |
| 16 | +++++ | 93 | +++ | 170 | +++ | 247 | + |
| 17 | ++++ | 94 | ++++ | 171 | + | 248 | +++ |
| 18 | ++++ | 95 | ++ | 172 | ++++ | 249 | + |
| 19 | ++++ | 96 | + | 173 | ++++ | 250 | + |
| 20 | +++++ | 97 | +++ | 174 | + | 251 | ++++ |
| 21 | ++++ | 98 | + | 175 | ++++ | 252 | + |
| 22 | ++++ | 99 | + | 176 | + | 253 | +++ |
| 23 | +++++ | 100 | ++++ | 177 | ++++ | 254 | + |
| 24 | +++++ | 101 | ++++ | 178 | + | 255 | ++++ |
| 25 | ++++ | 102 | +++ | 179 | ++++ | 256 | ++++ |
| 26 | ++++ | 103 | ++++ | 180 | ++++ | 257 | + |
| 27 | ++++ | 104 | ++++ | 181 | +++ | 258 | + |
| 28 | +++ | 105 | + | 182 | + | 259 | +++ |
| 29 | ++++ | 106 | + | 183 | +++ | 260 | + |
| 30 | + | 107 | ++++ | 184 | ++++ | 261 | + |
| 31 | + | 108 | ++++ | 185 | + | 262 | + |
| 32 | + | 109 | + | 186 | ++ | 263 | ++++ |
| 33 | ++++ | 110 | ++++ | 187 | ++++ | 264 | + |
| 34 | +++ | 111 | + | 188 | + | 265 | ++++ |
| 35 | + | 112 | ++++ | 189 | ++++ | 266 | + |
| 36 | ++++ | 113 | ++ | 190 | ++++ | 267 | + |
| 37 | ++++ | 114 | ++++ | 191 | +++ | 268 | +++ |
| 38 | + | 115 | ++++ | 192 | + | 269 | + |
| 39 | + | 116 | +++ | 193 | ++++ | 270 | + |
| 40 | ++++ | 117 | + | 194 | ++++ | 271 | + |
| 41 | +++ | 118 | ++++ | 195 | +++ | 272 | ++++ |
| 42 | +++++ | 119 | ++++ | 196 | + | 273 | + |
| 43 | ++++ | 120 | + | 197 | ++++ | 274 | +++++ |
| 44 | +++ | 121 | +++ | 198 | +++ | 275 | ++ |
| 45 | + | 122 | +++ | 199 | + | 276 | + |
| 46 | + | 123 | ++++ | 200 | ++++ | 277 | +++ |
| 47 | +++++ | 124 | + | 201 | + | 278 | + |
| 48 | + | 125 | ++++ | 202 | +++ | 279 | ++++ |
| 49 | + | 126 | ++++ | 203 | + | 280 | + |
| 50 | + | 127 | +++ | 204 | ++++ | 281 | ++++ |
| 51 | + | 128 | + | 205 | +++ | 282 | ++++ |
| 52 | + | 129 | ++++ | 206 | ++++ | 283 | ++++ |
| 53 | + | 130 | ++++ | 207 | + | 284 | +++++ |
| 54 | + | 131 | + | 208 | ++++ | 285 | + |
| 55 | +++ | 132 | ++ | 209 | + | 286 | +++ |
| 56 | + | 133 | ++++ | 210 | + | 287 | ++++ |
| 57 | + | 134 | +++ | 211 | +++ | 288 | +++ |
| 58 | + | 135 | ++++ | 212 | +++ | 289 | +++++ |
| 59 | ++++ | 136 | +++ | 213 | ++++ | 290 | ++++ |
| 60 | +++ | 137 | + | 214 | ++ | 291 | ++++ |
| 61 | ++++ | 138 | ++++ | 215 | + | 292 | +++++ |
| 62 | + | 139 | ++++ | 216 | ++++ | 293 | ++ |
| 63 | ++++ | 140 | +++ | 217 | + | 294 | ++++ |
| 64 | ++++ | 141 | + | 218 | ++++ | 295 | +++ |
| 65 | + | 142 | ++++ | 219 | ++++ | 296 | + |
| 66 | + | 143 | + | 220 | + | 297 | ++++ |
| 67 | +++ | 144 | ++++ | 221 | +++ | 298 | +++ |
| 68 | + | 145 | + | 222 | + | 299 | + |
| 69 | +++ | 146 | + | 223 | + | 300 | ++ |
| 70 | ++++ | 147 | + | 224 | +++ | 301 | ++++ |
| 71 | ++++ | 148 | ++++ | 225 | ++++ | 302 | + |
| 72 | ++++ | 149 | + | 226 | + | 303 | ++ |
| 73 | ++++ | 150 | + | 227 | +++ | 304 | + |
| 74 | + | 151 | ++++ | 228 | + | 305 | +++ |
| 75 | +++ | 152 | ++++ | 229 | ++++ | 306 | + |
| 76 | ++++ | 153 | ++ | 230 | ++++ | | |
| 77 | ++++ | 154 | +++ | 231 | ++++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 8:

In a 10 mL reaction bottle, 306 imine reductase enzyme solutions (a mass ratio of substrate ketone to imine reductase in the enzyme solution was 1:8) shown in Table 1 were respectively added, then 6.72 mg of GDH, 21.6 mg of Glu, 1.33 mg of NAD⁺, 1.53 mg of NADP⁺, 6.72 mg (20 mM) of 1-(tetrahydro-2H-pyran-4-yl)ethanone 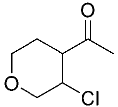 (dissolved in 10 µL of DMSO), and 10 mg (100 mM) of hydrazine hydrate were added. The total volume was adjusted to 2 mL with 100 mM Tris-HCI buffer (pH 8.0), and the reaction was performed for 16 h at 30 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 9 below.

**Table 9:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | +++ | 155 | + | 232 | ++ |
| 2 | + | 79 | ++++ | 156 | ++++ | 233 | + |
| 3 | + | 80 | ++++ | 157 | + | 234 | +++ |
| 4 | + | 81 | +++ | 158 | ++++ | 235 | ++++ |
| 5 | +++ | 82 | + | 159 | + | 236 | +++ |
| 6 | + | 83 | + | 160 | + | 237 | + |
| 7 | ++ | 84 | +++ | 161 | ++++ | 238 | + |
| 8 | +++ | 85 | ++++ | 162 | ++++ | 239 | +++ |
| 9 | +++ | 86 | +++ | 163 | + | 240 | ++++ |
| 10 | + | 87 | ++++ | 164 | ++++ | 241 | + |
| 11 | + | 88 | +++ | 165 | + | 242 | ++++ |
| 12 | ++++ | 89 | +++ | 166 | +++ | 243 | + |
| 13 | + | 90 | +++ | 167 | +++ | 244 | ++++ |
| 14 | ++++ | 91 | +++ | 168 | ++++ | 245 | + |
| 15 | +++ | 92 | +++ | 169 | + | 246 | + |
| 16 | +++++ | 93 | +++ | 170 | +++ | 247 | + |
| 17 | ++++ | 94 | ++++ | 171 | + | 248 | +++ |
| 18 | + | 95 | ++ | 172 | ++++ | 249 | + |
| 19 | + | 96 | + | 173 | ++++ | 250 | + |
| 20 | +++++ | 97 | +++ | 174 | + | 251 | ++++ |
| 21 | + | 98 | + | 175 | ++++ | 252 | + |
| 22 | ++++ | 99 | + | 176 | + | 253 | +++ |
| 23 | + | 100 | ++++ | 177 | +++ | 254 | + |
| 24 | +++++ | 101 | ++++ | 178 | + | 255 | ++++ |
| 25 | ++++ | 102 | +++ | 179 | ++++ | 256 | +++ |
| 26 | ++++ | 103 | +++ | 180 | ++++ | 257 | + |
| 27 | + | 104 | ++++ | 181 | +++ | 258 | + |
| 28 | +++ | 105 | + | 182 | + | 259 | +++ |
| 29 | + | 106 | + | 183 | +++ | 260 | + |
| 30 | + | 107 | ++++ | 184 | ++++ | 261 | + |
| 31 | + | 108 | ++++ | 185 | + | 262 | + |
| 32 | + | 109 | + | 186 | ++ | 263 | ++++ |
| 33 | ++++ | 110 | ++++ | 187 | ++++ | 264 | + |
| 34 | +++ | 111 | + | 188 | + | 265 | + |
| 35 | + | 112 | ++++ | 189 | +++ | 266 | + |
| 36 | ++++ | 113 | ++ | 190 | ++++ | 267 | + |
| 37 | + | 114 | ++++ | 191 | +++ | 268 | +++ |
| 38 | + | 115 | ++++ | 192 | + | 269 | + |
| 39 | + | 116 | +++ | 193 | ++++ | 270 | + |
| 40 | + | 117 | + | 194 | +++ | 271 | + |
| 41 | +++ | 118 | +++ | 195 | +++ | 272 | ++++ |
| 42 | + | 119 | +++ | 196 | + | 273 | + |
| 43 | ++++ | 120 | + | 197 | ++++ | 274 | +++ |
| 44 | +++ | 121 | +++ | 198 | +++ | 275 | ++ |
| 45 | + | 122 | +++ | 199 | + | 276 | + |
| 46 | + | 123 | +++ | 200 | ++++ | 277 | + |
| 47 | + | 124 | + | 201 | + | 278 | + |
| 48 | + | 125 | + | 202 | +++ | 279 | ++++ |
| 49 | +++ | 126 | ++++ | 203 | + | 280 | + |
| 50 | + | 127 | +++ | 204 | ++++ | 281 | + |
| 51 | + | 128 | + | 205 | +++ | 282 | + |
| 52 | + | 129 | + | 206 | ++++ | 283 | ++++ |
| 53 | + | 130 | ++++ | 207 | + | 284 | +++ |
| 54 | + | 131 | + | 208 | +++ | 285 | + |
| 55 | +++ | 132 | ++ | 209 | + | 286 | +++ |
| 56 | + | 133 | +++ | 210 | + | 287 | ++++ |
| 57 | + | 134 | +++ | 211 | +++ | 288 | + |
| 58 | + | 135 | ++++ | 212 | +++ | 289 | +++++ |
| 59 | ++++ | 136 | +++ | 213 | ++++ | 290 | +++ |
| 60 | +++ | 137 | + | 214 | ++ | 291 | + |
| 61 | +++ | 138 | ++++ | 215 | + | 292 | +++++ |
| 62 | + | 139 | ++++ | 216 | ++++ | 293 | ++ |
| 63 | ++++ | 140 | +++ | 217 | + | 294 | ++++ |
| 64 | +++ | 141 | + | 218 | ++++ | 295 | +++ |
| 65 | + | 142 | +++ | 219 | +++ | 296 | + |
| 66 | + | 143 | + | 220 | + | 297 | ++++ |
| 67 | +++ | 144 | ++++ | 221 | +++ | 298 | +++ |
| 68 | + | 145 | + | 222 | + | 299 | + |
| 69 | +++ | 146 | + | 223 | + | 300 | ++ |
| 70 | ++++ | 147 | + | 224 | +++ | 301 | +++ |
| 71 | +++ | 148 | ++++ | 225 | ++++ | 302 | + |
| 72 | +++ | 149 | + | 226 | + | 303 | ++ |
| 73 | +++ | 150 | + | 227 | +++ | 304 | + |
| 74 | + | 151 | +++ | 228 | + | 305 | +++ |
| 75 | +++ | 152 | ++++ | 229 | ++++ | 306 | + |
| 76 | ++++ | 153 | ++ | 230 | + | | |
| 77 | +++ | 154 | +++ | 231 | +++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 9:

In a 10 mL reaction bottle, 306 imine reductase enzyme solutions (a mass ratio of substrate ketone to imine reductase in the enzyme solution was 1:8) shown in Table 1 were respectively added, then 4 mg of GDH, 21.6 mg of Glu, 1.33 mg of NAD⁺, 1.53 mg of NADP⁺, 4 mg (20 mM) of 1-(tetrahydro-2H-pyran-4-yl)ethanone 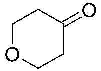 (dissolved in 10 µL of DMSO), and 10 mg (100 mM) of hydrazine hydrate were added. The total volume was adjusted to 2 mL with 100 mM Tris-HCl buffer (pH 9.0), and the reaction was performed for 16 h at 30 ^{o}C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 10 below.

**Table 10:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | ++ |
| 2 | +++ | 79 | ++++ | 156 | ++++ | 233 | + |
| 3 | +++ | 80 | ++++ | 157 | + | 234 | +++ |
| 4 | ++++ | 81 | +++ | 158 | ++++ | 235 | ++++ |
| 5 | ++++ | 82 | + | 159 | + | 236 | ++++ |
| 6 | + | 83 | + | 160 | + | 237 | + |
| 7 | ++++ | 84 | + | 161 | ++++ | 238 | + |
| 8 | ++ | 85 | ++++ | 162 | ++++ | 239 | +++ |
| 9 | +++ | 86 | ++++ | 163 | + | 240 | ++++ |
| 10 | + | 87 | ++++ | 164 | ++++ | 241 | + |
| 11 | + | 88 | +++ | 165 | + | 242 | + |
| 12 | ++ | 89 | ++++ | 166 | +++ | 243 | + |
| 13 | ++ | 90 | +++ | 167 | +++ | 244 | ++ |
| 14 | ++++ | 91 | +++ | 168 | ++++ | 245 | ++ |
| 15 | +++ | 92 | +++ | 169 | + | 246 | + |
| 16 | +++++ | 93 | + | 170 | +++ | 247 | + |
| 17 | + | 94 | ++++ | 171 | + | 248 | +++ |
| 18 | ++++ | 95 | ++ | 172 | ++++ | 249 | + |
| 19 | ++++ | 96 | + | 173 | ++++ | 250 | + |
| 20 | +++++ | 97 | +++ | 174 | + | 251 | ++ |
| 21 | ++++ | 98 | + | 175 | ++++ | 252 | + |
| 22 | ++++ | 99 | + | 176 | + | 253 | +++ |
| 23 | +++++ | 100 | ++++ | 177 | ++++ | 254 | + |
| 24 | +++++ | 101 | ++++ | 178 | + | 255 | ++++ |
| 25 | +++ | 102 | +++ | 179 | +++ | 256 | ++++ |
| 26 | ++++ | 103 | ++++ | 180 | ++++ | 257 | + |
| 27 | ++++ | 104 | ++++ | 181 | +++ | 258 | + |
| 28 | ++++ | 105 | + | 182 | + | 259 | + |
| 29 | ++++ | 106 | + | 183 | +++ | 260 | + |
| 30 | + | 107 | + | 184 | ++++ | 261 | + |
| 31 | ++++ | 108 | ++++ | 185 | + | 262 | + |
| 32 | + | 109 | + | 186 | ++ | 263 | + |
| 33 | ++++ | 110 | ++++ | 187 | ++++ | 264 | + |
| 34 | +++ | 111 | + | 188 | + | 265 | ++ |
| 35 | + | 112 | ++++ | 189 | +++ | 266 | + |
| 36 | +++ | 113 | ++ | 190 | ++++ | 267 | + |
| 37 | ++++ | 114 | ++++ | 191 | +++ | 268 | +++ |
| 38 | + | 115 | ++++ | 192 | + | 269 | + |
| 39 | + | 116 | +++ | 193 | +++ | 270 | + |
| 40 | ++++ | 117 | + | 194 | ++++ | 271 | + |
| 41 | ++++ | 118 | + | 195 | +++ | 272 | ++++ |
| 42 | ++++ | 119 | ++++ | 196 | + | 273 | + |
| 43 | ++++ | 120 | + | 197 | ++++ | 274 | +++++ |
| 44 | +++ | 121 | +++ | 198 | +++ | 275 | ++ |
| 45 | + | 122 | +++ | 199 | + | 276 | + |
| 46 | + | 123 | ++++ | 200 | ++++ | 277 | +++ |
| 47 | +++++ | 124 | + | 201 | + | 278 | + |
| 48 | + | 125 | + | 202 | +++ | 279 | ++++ |
| 49 | + | 126 | ++++ | 203 | + | 280 | + |
| 50 | + | 127 | +++++ | 204 | ++++ | 281 | ++ |
| 51 | + | 128 | + | 205 | +++ | 282 | ++++ |
| 52 | + | 129 | ++++ | 206 | ++++ | 283 | ++++ |
| 53 | +++++ | 130 | ++++ | 207 | + | 284 | +++++ |
| 54 | + | 131 | + | 208 | ++++ | 285 | + |
| 55 | + | 132 | ++ | 209 | + | 286 | +++ |
| 56 | + | 133 | ++++ | 210 | + | 287 | ++ |
| 57 | + | 134 | +++ | 211 | +++ | 288 | +++ |
| 58 | + | 135 | ++++ | 212 | +++ | 289 | +++ |
| 59 | + | 136 | +++ | 213 | ++++ | 290 | +++ |
| 60 | +++ | 137 | + | 214 | ++ | 291 | ++ |
| 61 | ++++ | 138 | +++ | 215 | + | 292 | ++ |
| 62 | + | 139 | ++++ | 216 | ++++ | 293 | ++ |
| 63 | ++++ | 140 | +++ | 217 | + | 294 | ++++ |
| 64 | ++++ | 141 | + | 218 | ++++ | 295 | +++ |
| 65 | + | 142 | ++++ | 219 | ++++ | 296 | + |
| 66 | + | 143 | + | 220 | + | 297 | ++++ |
| 67 | ++++ | 144 | ++++ | 221 | +++ | 298 | +++ |
| 68 | + | 145 | + | 222 | + | 299 | ++ |
| 69 | +++ | 146 | + | 223 | + | 300 | ++ |
| 70 | + | 147 | + | 224 | +++ | 301 | ++++ |
| 71 | ++++ | 148 | ++++ | 225 | ++++ | 302 | + |
| 72 | +++++ | 149 | + | 226 | + | 303 | ++ |
| 73 | ++++ | 150 | + | 227 | +++ | 304 | + |
| 74 | + | 151 | +++ | 228 | + | 305 | +++ |
| 75 | ++++ | 152 | ++++ | 229 | ++++ | 306 | ++ |
| 76 | ++++ | 153 | ++ | 230 | ++++ | | |
| 77 | ++++ | 154 | +++ | 231 | ++++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 10:

In a 10 mL reaction bottle, 306 imine reductase enzyme solutions (a mass ratio of substrate ketone to imine reductase in the enzyme solution was 1:5) shown in Table 1 were respectively added, then 13.44 mg of GDH, 21.6 mg of Glu, 1.33 mg of NAD⁺, 1.53 mg of NADP⁺, 13.44 mg (60 mM) of 1-(tetrahydro-2H-pyran-4-yl)ethanone 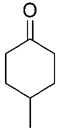 (dissolved in 10 µL of DMSO), and 10 mg (100 mM) of hydrazine hydrate were added. The total volume was adjusted to 2 mL with 100 mM Tris-HCI buffer (pH 9.0), and the reaction was performed for 16 h at 30 °C through shaking. 500 µL of the system was taken, the reaction was terminated with 100 µL of a 0.1 M sodium hydroxide solution, full shaking and well mixing were performed, extraction was performed using ethyl acetate, and then GC or LC-MS detection was performed to determine a conversion rate, as shown in Table 11 below.

**Table 11:**

| Enzyme number | Conversion rate | Enzyme number | Conversion rate | Enzyme number | Conversion rate | Enzyme number | Conversion rate |
|---|---|---|---|---|---|---|---|
| 1 | ++ | 78 | ++ | 155 | + | 232 | ++ |
| 2 | + | 79 | + | 156 | + | 233 | ++ |
| 3 | + | 80 | + | 157 | + | 234 | + |
| 4 | ++++ | 81 | + | 158 | + | 235 | + |
| 5 | +++ | 82 | + | 159 | + | 236 | + |
| 6 | + | 83 | ++ | 160 | ++++ | 237 | + |
| 7 | + | 84 | ++ | 161 | ++++ | 238 | + |
| 8 | + | 85 | + | 162 | + | 239 | ++++ |
| 9 | +++ | 86 | + | 163 | + | 240 | + |
| 10 | + | 87 | +++ | 164 | + | 241 | + |
| 11 | ++ | 88 | ++ | 165 | ++ | 242 | + |
| 12 | + | 89 | +++ | 166 | + | 243 | + |
| 13 | ++ | 90 | + | 167 | ++ | 244 | + |
| 14 | + | 91 | + | 168 | + | 245 | + |
| 15 | + | 92 | + | 169 | + | 246 | + |
| 16 | +++++ | 93 | +++ | 170 | + | 247 | + |
| 17 | +++++ | 94 | +++ | 171 | + | 248 | + |
| 18 | +++++ | 95 | ++ | 172 | + | 249 | + |
| 19 | +++ | 96 | ++ | 173 | + | 250 | + |
| 20 | +++++ | 97 | + | 174 | + | 251 | ++++ |
| 21 | ++++ | 98 | + | 175 | +++++ | 252 | + |
| 22 | ++++ | 99 | + | 176 | ++++ | 253 | + |
| 23 | +++++ | 100 | +++ | 177 | + | 254 | + |
| 24 | +++++ | 101 | ++ | 178 | + | 255 | + |
| 25 | + | 102 | + | 179 | + | 256 | + |
| 26 | +++ | 103 | ++ | 180 | + | 257 | ++ |
| 27 | ++++ | 104 | + | 181 | + | 258 | ++ |
| 28 | ++ | 105 | ++++ | 182 | + | 259 | + |
| 29 | ++ | 106 | +++ | 183 | + | 260 | + |
| 30 | + | 107 | +++++ | 184 | +++++ | 261 | + |
| 31 | ++++ | 108 | + | 185 | + | 262 | + |
| 32 | ++ | 109 | ++++ | 186 | ++++ | 263 | + |
| 33 | ++ | 110 | +++ | 187 | ++++ | 264 | + |
| 34 | + | 111 | + | 188 | + | 265 | + |
| 35 | ++ | 112 | + | 189 | + | 266 | +++++ |
| 36 | + | 113 | + | 190 | ++ | 267 | + |
| 37 | +++++ | 114 | + | 191 | ++ | 268 | + |
| 38 | + | 115 | +++++ | 192 | + | 269 | + |
| 39 | + | 116 | ++++ | 193 | + | 270 | + |
| 40 | + | 117 | + | 194 | + | 271 | + |
| 41 | +++++ | 118 | ++++ | 195 | + | 272 | + |
| 42 | +++ | 119 | + | 196 | + | 273 | ++++ |
| 43 | + | 120 | + | 197 | + | 274 | + |
| 44 | + | 121 | + | 198 | + | 275 | ++++ |
| 45 | + | 122 | +++++ | 199 | + | 276 | + |
| 46 | + | 123 | + | 200 | ++ | 277 | + |
| 47 | +++++ | 124 | + | 201 | + | 278 | + |
| 48 | ++ | 125 | + | 202 | + | 279 | + |
| 49 | ++ | 126 | + | 203 | + | 280 | + |
| 50 | + | 127 | +++++ | 204 | + | 281 | + |
| 51 | + | 128 | + | 205 | ++++ | 282 | + |
| 52 | + | 129 | + | 206 | + | 283 | + |
| 53 | + | 130 | ++++ | 207 | ++++ | 284 | +++++ |
| 54 | + | 131 | + | 208 | + | 285 | ++++ |
| 55 | + | 132 | + | 209 | + | 286 | + |
| 56 | + | 133 | + | 210 | + | 287 | + |
| 57 | ++ | 134 | + | 211 | + | 288 | + |
| 58 | ++ | 135 | +++++ | 212 | + | 289 | + |
| 59 | ++++ | 136 | +++++ | 213 | + | 290 | + |
| 60 | ++ | 137 | + | 214 | + | 291 | + |
| 61 | + | 138 | + | 215 | + | 292 | + |
| 62 | ++ | 139 | ++++ | 216 | + | 293 | + |
| 63 | ++ | 140 | + | 217 | +++++ | 294 | ++++ |
| 64 | + | 141 | + | 218 | + | 295 | + |
| 65 | + | 142 | + | 219 | ++++ | 296 | + |
| 66 | + | 143 | + | 220 | + | 297 | + |
| 67 | + | 144 | + | 221 | + | 298 | + |
| 68 | + | 145 | + | 222 | + | 299 | + |
| 69 | + | 146 | + | 223 | + | 300 | ++++ |
| 70 | + | 147 | +++++ | 224 | ++ | 301 | + |
| 71 | ++ | 148 | ++++ | 225 | + | 302 | + |
| 72 | ++++ | 149 | ++++ | 226 | + | 303 | + |
| 73 | ++ | 150 | + | 227 | + | 304 | +++++ |
| 74 | + | 151 | + | 228 | + | 305 | + |
| 75 | +++ | 152 | + | 229 | + | 306 | + |
| 76 | +++++ | 153 | +++++ | 230 | ++ | | |
| 77 | ++ | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 11 :

In a 10 mL reaction bottle, 306 imine reductase enzyme solutions (a mass ratio of substrate ketone to imine reductase in the enzyme solution was 1:5) shown in Table 1 were respectively added, then 19.6 mg of GDH, 21.6 mg of Glu, 1.33 mg of NAD⁺, 1.53 mg of NADP⁺, 19.6 mg (100 mM) of cyclohexanone 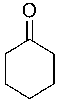 (dissolved in 10 µL of DMSO), and 52.8 mg of tert butoxycarbonyl hydrazine (200 mM) were added. The total volume was adjusted to 2 mL with 100 mM Tris-HCI buffer (pH 9.0), and the reaction was performed for 18 h at 30 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 12 below.

**Table 12:**

| Enzyme number | Conversion rate | Enzyme number | Conversion rate | Enzyme number | Conversion rate | Enzyme number | Conversion rate |
|---|---|---|---|---|---|---|---|
| 1 | +++++ | 78 | + | 155 | + | 232 | + |
| 2 | + | 79 | + | 156 | + | 233 | + |
| 3 | +++++ | 80 | + | 157 | + | 234 | ++ |
| 4 | + | 81 | + | 158 | + | 235 | + |
| 5 | +++++ | 82 | + | 159 | + | 236 | + |
| 6 | + | 83 | + | 160 | +++ | 237 | + |
| 7 | + | 84 | + | 161 | + | 238 | ++++ |
| 8 | + | 85 | + | 162 | + | 239 | + |
| 9 | ++ | 86 | + | 163 | + | 240 | + |
| 10 | +++++ | 87 | +++++ | 164 | + | 241 | + |
| 11 | + | 88 | + | 165 | + | 242 | + |
| 12 | + | 89 | + | 166 | + | 243 | + |
| 13 | ++++ | 90 | +++++ | 167 | + | 244 | + |
| 14 | ++ | 91 | + | 168 | + | 245 | + |
| 15 | + | 92 | ++ | 169 | + | 246 | + |
| 16 | + | 93 | ++ | 170 | + | 247 | + |
| 17 | + | 94 | +++++ | 171 | + | 248 | ++++ |
| 18 | + | 95 | + | 172 | + | 249 | + |
| 19 | ++ | 96 | + | 173 | + | 250 | + |
| 20 | + | 97 | + | 174 | + | 251 | + |
| 21 | + | 98 | + | 175 | +++++ | 252 | + |
| 22 | +++++ | 99 | + | 176 | + | 253 | + |
| 23 | + | 100 | +++++ | 177 | + | 254 | + |
| 24 | +++++ | 101 | ++ | 178 | + | 255 | + |
| 25 | +++++ | 102 | ++ | 179 | + | 256 | + |
| 26 | ++++ | 103 | +++++ | 180 | + | 257 | + |
| 27 | +++++ | 104 | +++ | 181 | + | 258 | + |
| 28 | +++++ | 105 | + | 182 | ++++ | 259 | + |
| 29 | +++++ | 106 | +++++ | 183 | + | 260 | ++ |
| 30 | + | 107 | + | 184 | +++ | 261 | + |
| 31 | ++++ | 108 | +++++ | 185 | + | 262 | + |
| 32 | + | 109 | + | 186 | ++++ | 263 | + |
| 33 | + | 110 | + | 187 | + | 264 | + |
| 34 | +++++ | 111 | + | 188 | + | 265 | + |
| 35 | + | 112 | + | 189 | + | 266 | + |
| 36 | ++ | 113 | + | 190 | + | 267 | + |
| 37 | +++++ | 114 | + | 191 | + | 268 | +++ |
| 38 | + | 115 | +++ | 192 | + | 269 | + |
| 39 | + | 116 | + | 193 | + | 270 | + |
| 40 | ++++ | 117 | + | 194 | ++ | 271 | + |
| 41 | +++++ | 118 | + | 195 | + | 272 | ++ |
| 42 | + | 119 | + | 196 | + | 273 | + |
| 43 | + | 120 | + | 197 | + | 274 | + |
| 44 | + | 121 | +++++ | 198 | + | 275 | + |
| 45 | + | 122 | +++++ | 199 | + | 276 | + |
| 46 | +++ | 123 | + | 200 | + | 277 | + |
| 47 | + | 124 | + | 201 | + | 278 | ++++ |
| 48 | + | 125 | + | 202 | + | 279 | + |
| 49 | + | 126 | + | 203 | +++++ | 280 | ++ |
| 50 | + | 127 | + | 204 | ++++ | 281 | + |
| 51 | + | 128 | + | 205 | + | 282 | + |
| 52 | + | 129 | + | 206 | + | 283 | + |
| 53 | + | 130 | +++ | 207 | +++ | 284 | + |
| 54 | + | 131 | + | 208 | + | 285 | + |
| 55 | + | 132 | + | 209 | + | 286 | + |
| 56 | + | 133 | + | 210 | + | 287 | + |
| 57 | + | 134 | + | 211 | + | 288 | + |
| 58 | + | 135 | + | 212 | + | 289 | + |
| 59 | + | 136 | + | 213 | + | 290 | ++++ |
| 60 | + | 137 | + | 214 | + | 291 | + |
| 61 | + | 138 | + | 215 | + | 292 | + |
| 62 | + | 139 | + | 216 | + | 293 | + |
| 63 | ++++ | 140 | + | 217 | +++ | 294 | + |
| 64 | + | 141 | + | 218 | + | 295 | + |
| 65 | + | 142 | +++++ | 219 | + | 296 | + |
| 66 | + | 143 | + | 220 | + | 297 | +++++ |
| 67 | + | 144 | + | 221 | ++ | 298 | + |
| 68 | + | 145 | + | 222 | + | 299 | + |
| 69 | + | 146 | + | 223 | + | 300 | ++++ |
| 70 | + | 147 | +++ | 224 | + | 301 | ++ |
| 71 | + | 148 | + | 225 | + | 302 | + |
| 72 | + | 149 | + | 226 | + | 303 | +++++ |
| 73 | + | 150 | + | 227 | +++++ | 304 | ++++ |
| 74 | + | 151 | +++++ | 228 | + | 305 | ++ |
| 75 | + | 152 | + | 229 | + | 306 | + |
| 76 | +++++ | 153 | + | 230 | + | | |
| 77 | + | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 12:

In a 10 mL reaction bottle, 306 imine reductase enzyme solutions (a mass ratio of substrate ketone to imine reductase in the enzyme solution was 1:5) shown in Table 1 were respectively added, then 19.6 mg of GDH, 21.6 mg of Glu, 1.33 mg of NAD⁺, 1.53 mg of NADP⁺, 19.6 mg (100 mM) of cyclohexanone 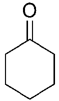 (dissolved in 10 µL of DMSO), and 66.4 mg of benzyloxycarbonyl hydrazine (200 mM) were added. The total volume was adjusted to 2 mL with 100 mM Tris-HCI buffer (pH 9.0), and the reaction was performed for 18 h at 30 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 13 below.

**Table 13:**

| Enzyme number | Conversion rate | Enzyme number | Conversion rate | Enzyme number | Conversion rate | Enzyme number | Conversion rate |
|---|---|---|---|---|---|---|---|
| 1 | +++ | 78 | + | 155 | + | 232 | + |
| 2 | + | 79 | + | 156 | + | 233 | + |
| 3 | ++++ | 80 | + | 157 | + | 234 | + |
| 4 | + | 81 | ++ | 158 | + | 235 | + |
| 5 | ++++ | 82 | + | 159 | + | 236 | + |
| 6 | ++ | 83 | + | 160 | + | 237 | + |
| 7 | + | 84 | + | 161 | + | 238 | + |
| 8 | + | 85 | + | 162 | + | 239 | + |
| 9 | + | 86 | ++ | 163 | + | 240 | + |
| 10 | +++ | 87 | + | 164 | + | 241 | +++ |
| 11 | + | 88 | + | 165 | + | 242 | + |
| 12 | + | 89 | + | 166 | + | 243 | + |
| 13 | + | 90 | + | 167 | + | 244 | + |
| 14 | + | 91 | ++ | 168 | + | 245 | + |
| 15 | + | 92 | + | 169 | + | 246 | + |
| 16 | + | 93 | + | 170 | + | 247 | + |
| 17 | + | 94 | +++ | 171 | +++ | 248 | ++ |
| 18 | + | 95 | + | 172 | + | 249 | + |
| 19 | ++ | 96 | + | 173 | + | 250 | + |
| 20 | ++ | 97 | + | 174 | + | 251 | + |
| 21 | ++++ | 98 | + | 175 | + | 252 | + |
| 22 | +++ | 99 | + | 176 | + | 253 | + |
| 23 | ++ | 100 | ++ | 177 | + | 254 | + |
| 24 | +++ | 101 | ++ | 178 | + | 255 | + |
| 25 | ++++ | 102 | + | 179 | + | 256 | + |
| 26 | ++ | 103 | +++ | 180 | + | 257 | + |
| 27 | ++ | 104 | + | 181 | + | 258 | + |
| 28 | + | 105 | +++ | 182 | ++ | 259 | + |
| 29 | + | 106 | + | 183 | + | 260 | + |
| 30 | + | 107 | + | 184 | + | 261 | + |
| 31 | + | 108 | + | 185 | + | 262 | + |
| 32 | + | 109 | + | 186 | + | 263 | + |
| 33 | + | 110 | + | 187 | + | 264 | + |
| 34 | ++ | 111 | + | 188 | + | 265 | + |
| 35 | + | 112 | + | 189 | + | 266 | + |
| 36 | + | 113 | + | 190 | + | 267 | + |
| 37 | ++ | 114 | + | 191 | + | 268 | + |
| 38 | ++ | 115 | + | 192 | ++++ | 269 | +++ |
| 39 | + | 116 | + | 193 | + | 270 | + |
| 40 | ++ | 117 | + | 194 | + | 271 | + |
| 41 | + | 118 | ++++ | 195 | + | 272 | + |
| 42 | + | 119 | + | 196 | + | 273 | + |
| 43 | + | 120 | + | 197 | + | 274 | + |
| 44 | + | 121 | + | 198 | + | 275 | + |
| 45 | + | 122 | + | 199 | + | 276 | + |
| 46 | + | 123 | + | 200 | + | 277 | + |
| 47 | + | 124 | + | 201 | + | 278 | ++ |
| 48 | + | 125 | +++ | 202 | + | 279 | + |
| 49 | + | 126 | + | 203 | + | 280 | + |
| 50 | + | 127 | + | 204 | + | 281 | + |
| 51 | + | 128 | + | 205 | + | 282 | + |
| 52 | + | 129 | + | 206 | + | 283 | + |
| 53 | + | 130 | + | 207 | + | 284 | + |
| 54 | + | 131 | + | 208 | + | 285 | ++++ |
| 55 | + | 132 | + | 209 | + | 286 | + |
| 56 | + | 133 | + | 210 | + | 287 | + |
| 57 | + | 134 | + | 211 | + | 288 | + |
| 58 | + | 135 | + | 212 | + | 289 | + |
| 59 | + | 136 | + | 213 | +++ | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | ++ | 140 | + | 217 | + | 294 | + |
| 64 | + | 141 | + | 218 | + | 295 | + |
| 65 | + | 142 | ++ | 219 | + | 296 | + |
| 66 | + | 143 | + | 220 | + | 297 | + |
| 67 | + | 144 | + | 221 | + | 298 | +++ |
| 68 | + | 145 | + | 222 | + | 299 | + |
| 69 | + | 146 | + | 223 | + | 300 | ++ |
| 70 | + | 147 | + | 224 | + | 301 | + |
| 71 | + | 148 | + | 225 | + | 302 | + |
| 72 | + | 149 | + | 226 | + | 303 | ++ |
| 73 | + | 150 | + | 227 | ++ | 304 | + |
| 74 | + | 151 | ++ | 228 | + | 305 | + |
| 75 | + | 152 | + | 229 | + | 306 | + |
| 76 | + | 153 | + | 230 | + | | |
| 77 | + | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 13:

In a 10 mL reaction bottle, 306 imine reductase enzyme solutions (a mass ratio of substrate ketone to imine reductase in the enzyme solution was 1:10) shown in Table 1 were respectively added, then 7.2 mg of GDH, 21.6 mg of Glu, 1.33 mg of NAD⁺, 1.53 mg of NADP⁺, 7.2 mg (50 mM) of 2-butanone 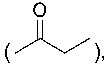 and 12 mg (120 mM) of hydrazine hydrate were added. The total volume was adjusted to 2 mL with 100 mM Tris-HCI buffer (pH 8.5), and the reaction was performed for 16 h at 37 °C through shaking. 500 µL of the system was taken, the reaction was terminated with 100 µL of a 0.1 M sodium hydroxide solution, full shaking and well mixing were performed, then centrifugation was performed for 10 min at 12000 rpm, and then GC or LC-MS detection was performed to determine a conversion rate, as shown in Table 14 below.

**Table 14:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | ++ | 79 | + | 156 | + | 233 | + |
| 3 | + | 80 | + | 157 | + | 234 | + |
| 4 | + | 81 | + | 158 | +++ | 235 | + |
| 5 | +++ | 82 | + | 159 | ++ | 236 | + |
| 6 | + | 83 | + | 160 | + | 237 | + |
| 7 | + | 84 | + | 161 | +++ | 238 | + |
| 8 | + | 85 | + | 162 | + | 239 | ++ |
| 9 | ++ | 86 | + | 163 | + | 240 | + |
| 10 | + | 87 | + | 164 | + | 241 | + |
| 11 | + | 88 | + | 165 | ++ | 242 | + |
| 12 | + | 89 | + | 166 | + | 243 | + |
| 13 | + | 90 | + | 167 | + | 244 | +++ |
| 14 | + | 91 | + | 168 | ++ | 245 | + |
| 15 | ++ | 92 | + | 169 | ++ | 246 | + |
| 16 | + | 93 | +++ | 170 | + | 247 | + |
| 17 | ++ | 94 | + | 171 | + | 248 | + |
| 18 | ++ | 95 | + | 172 | + | 249 | + |
| 19 | + | 96 | + | 173 | + | 250 | + |
| 20 | ++ | 97 | + | 174 | + | 251 | + |
| 21 | + | 98 | + | 175 | + | 252 | + |
| 22 | + | 99 | + | 176 | + | 253 | + |
| 23 | + | 100 | + | 177 | + | 254 | ++ |
| 24 | +++ | 101 | + | 178 | + | 255 | + |
| 25 | + | 102 | + | 179 | + | 256 | + |
| 26 | + | 103 | + | 180 | ++ | 257 | + |
| 27 | ++ | 104 | + | 181 | + | 258 | ++ |
| 28 | + | 105 | ++ | 182 | + | 259 | + |
| 29 | + | 106 | ++ | 183 | ++ | 260 | + |
| 30 | + | 107 | + | 184 | + | 261 | + |
| 31 | ++ | 108 | + | 185 | + | 262 | + |
| 32 | + | 109 | + | 186 | + | 263 | + |
| 33 | + | 110 | ++ | 187 | + | 264 | + |
| 34 | + | 111 | + | 188 | ++++ | 265 | + |
| 35 | + | 112 | + | 189 | ++ | 266 | + |
| 36 | + | 113 | + | 190 | +++ | 267 | + |
| 37 | ++++ | 114 | + | 191 | + | 268 | + |
| 38 | + | 115 | + | 192 | + | 269 | + |
| 39 | + | 116 | + | 193 | + | 270 | ++ |
| 40 | + | 117 | + | 194 | + | 271 | + |
| 41 | + | 118 | + | 195 | + | 272 | + |
| 42 | + | 119 | + | 196 | + | 273 | + |
| 43 | + | 120 | + | 197 | + | 274 | +++ |
| 44 | + | 121 | ++ | 198 | + | 275 | + |
| 45 | + | 122 | + | 199 | + | 276 | + |
| 46 | +++ | 123 | + | 200 | + | 277 | + |
| 47 | + | 124 | + | 201 | + | 278 | ++ |
| 48 | + | 125 | + | 202 | + | 279 | + |
| 49 | + | 126 | + | 203 | ++ | 280 | + |
| 50 | + | 127 | + | 204 | ++ | 281 | + |
| 51 | + | 128 | + | 205 | + | 282 | + |
| 52 | + | 129 | + | 206 | + | 283 | + |
| 53 | + | 130 | + | 207 | + | 284 | + |
| 54 | +++ | 131 | + | 208 | + | 285 | + |
| 55 | + | 132 | +++ | 209 | + | 286 | + |
| 56 | + | 133 | + | 210 | + | 287 | + |
| 57 | + | 134 | ++ | 211 | ++ | 288 | + |
| 58 | + | 135 | + | 212 | + | 289 | + |
| 59 | + | 136 | + | 213 | + | 290 | + |
| 60 | + | 137 | + | 214 | +++ | 291 | + |
| 61 | + | 138 | + | 215 | ++ | 292 | + |
| 62 | + | 139 | + | 216 | +++ | 293 | + |
| 63 | + | 140 | + | 217 | + | 294 | + |
| 64 | + | 141 | + | 218 | + | 295 | ++++ |
| 65 | + | 142 | + | 219 | + | 296 | + |
| 66 | + | 143 | + | 220 | + | 297 | ++ |
| 67 | + | 144 | + | 221 | + | 298 | + |
| 68 | + | 145 | + | 222 | + | 299 | + |
| 69 | + | 146 | + | 223 | + | 300 | + |
| 70 | + | 147 | ++ | 224 | + | 301 | + |
| 71 | + | 148 | ++ | 225 | + | 302 | + |
| 72 | +++ | 149 | ++ | 226 | + | 303 | + |
| 73 | + | 150 | + | 227 | + | 304 | + |
| 74 | + | 151 | + | 228 | + | 305 | + |
| 75 | + | 152 | + | 229 | ++ | 306 | ++ |
| 76 | + | 153 | + | 230 | + | | |
| 77 | + | 154 | + | 231 | ++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 14:

In a 10 mL reaction bottle, 306 imine reductase enzyme solutions (a mass ratio of substrate ketone to imine reductase in the enzyme solution was 1:10) shown in Table 1 were respectively added, then 4.8 mg of GDH, 21.6 mg of Glu, 1.33 mg of NAD⁺, 1.53 mg of NADP⁺, 4.8 mg (20 mM) of acetophenone 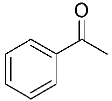 (dissolved in 10 µL of DMSO), and 20 mg (200 mM) of hydrazine hydrate were added. The total volume was adjusted to 2 mL with 100 mM Tris-HCI buffer (pH 8.5), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 15 below.

**Table 15:**

| Enzyme number | Conversion rate | Enzyme number | Conversion rate | Enzyme number | Conversion rate | Enzyme number | Conversion rate |
|---|---|---|---|---|---|---|---|
| 1 | +++ | 78 | + | 155 | + | 232 | + |
| 2 | +++++ | 79 | + | 156 | +++++ | 233 | +++++ |
| 3 | +++++ | 80 | + | 157 | + | 234 | +++++ |
| 4 | ++++ | 81 | +++++ | 158 | + | 235 | + |
| 5 | ++++ | 82 | + | 159 | +++ | 236 | + |
| 6 | +++ | 83 | + | 160 | +++ | 237 | + |
| 7 | +++ | 84 | +++++ | 161 | +++ | 238 | ++ |
| 8 | +++++ | 85 | + | 162 | + | 239 | ++ |
| 9 | + | 86 | + | 163 | + | 240 | + |
| 10 | ++++ | 87 | + | 164 | ++ | 241 | + |
| 11 | ++++ | 88 | +++++ | 165 | ++ | 242 | + |
| 12 | +++++ | 89 | + | 166 | + | 243 | + |
| 13 | ++++ | 90 | + | 167 | + | 244 | + |
| 14 | ++ | 91 | + | 168 | +++++ | 245 | ++++ |
| 15 | ++++ | 92 | + | 169 | ++ | 246 | + |
| 16 | ++++ | 93 | + | 170 | +++++ | 247 | + |
| 17 | ++++ | 94 | ++++ | 171 | +++ | 248 | + |
| 18 | + | 95 | ++++ | 172 | +++ | 249 | ++++ |
| 19 | ++++ | 96 | + | 173 | + | 250 | + |
| 20 | ++ | 97 | + | 174 | + | 251 | + |
| 21 | +++ | 98 | + | 175 | + | 252 | + |
| 22 | ++++ | 99 | + | 176 | + | 253 | + |
| 23 | +++ | 100 | ++++ | 177 | ++++ | 254 | + |
| 24 | +++ | 101 | +++++ | 178 | + | 255 | ++ |
| 25 | +++ | 102 | ++++ | 179 | ++ | 256 | ++ |
| 26 | +++++ | 103 | ++++ | 180 | ++ | 257 | + |
| 27 | ++ | 104 | +++ | 181 | + | 258 | + |
| 28 | +++ | 105 | + | 182 | + | 259 | + |
| 29 | +++++ | 106 | +++++ | 183 | +++ | 260 | +++++ |
| 30 | +++++ | 107 | +++ | 184 | + | 261 | +++++ |
| 31 | + | 108 | + | 185 | ++ | 262 | + |
| 32 | ++++ | 109 | ++ | 186 | ++ | 263 | + |
| 33 | ++++ | 110 | + | 187 | + | 264 | + |
| 34 | ++ | 111 | ++ | 188 | +++++ | 265 | + |
| 35 | + | 112 | + | 189 | +++++ | 266 | ++++ |
| 36 | ++++ | 113 | +++ | 190 | + | 267 | + |
| 37 | +++ | 114 | +++++ | 191 | + | 268 | + |
| 38 | ++++ | 115 | ++ | 192 | ++++ | 269 | + |
| 39 | ++++ | 116 | ++ | 193 | + | 270 | ++ |
| 40 | ++ | 117 | + | 194 | + | 271 | ++ |
| 41 | +++ | 118 | + | 195 | ++ | 272 | + |
| 42 | +++++ | 119 | +++++ | 196 | ++ | 273 | + |
| 43 | +++++ | 120 | +++ | 197 | ++++ | 274 | + |
| 44 | ++++ | 121 | +++ | 198 | + | 275 | + |
| 45 | ++++ | 122 | + | 199 | + | 276 | ++ |
| 46 | +++ | 123 | ++ | 200 | + | 277 | ++ |
| 47 | ++ | 124 | ++ | 201 | + | 278 | + |
| 48 | +++++ | 125 | ++ | 202 | + | 279 | +++++ |
| 49 | +++++ | 126 | + | 203 | ++ | 280 | ++++ |
| 50 | ++++ | 127 | +++ | 204 | ++ | 281 | +++++ |
| 51 | ++ | 128 | +++ | 205 | +++ | 282 | + |
| 52 | ++ | 129 | + | 206 | +++ | 283 | + |
| 53 | ++ | 130 | + | 207 | + | 284 | + |
| 54 | +++++ | 131 | + | 208 | +++ | 285 | + |
| 55 | ++++ | 132 | +++++ | 209 | + | 286 | ++ |
| 56 | +++++ | 133 | +++ | 210 | + | 287 | + |
| 57 | ++ | 134 | +++++ | 211 | + | 288 | ++ |
| 58 | ++ | 135 | +++++ | 212 | + | 289 | + |
| 59 | + | 136 | + | 213 | ++ | 290 | + |
| 60 | + | 137 | ++ | 214 | ++ | 291 | ++++ |
| 61 | + | 138 | + | 215 | + | 292 | + |
| 62 | + | 139 | + | 216 | +++++ | 293 | ++++ |
| 63 | + | 140 | + | 217 | + | 294 | ++++ |
| 64 | ++ | 141 | +++ | 218 | +++++ | 295 | + |
| 65 | ++ | 142 | ++++ | 219 | + | 296 | + |
| 66 | ++ | 143 | + | 220 | + | 297 | + |
| 67 | + | 144 | ++++ | 221 | + | 298 | + |
| 68 | + | 145 | + | 222 | + | 299 | + |
| 69 | + | 146 | + | 223 | + | 300 | + |
| 70 | ++ | 147 | +++ | 224 | ++ | 301 | ++++ |
| 71 | + | 148 | +++ | 225 | ++ | 302 | + |
| 72 | ++ | 149 | + | 226 | ++ | 303 | + |
| 73 | ++ | 150 | + | 227 | + | 304 | +++++ |
| 74 | + | 151 | ++ | 228 | + | 305 | + |
| 75 | +++++ | 152 | + | 229 | + | 306 | + |
| 76 | + | 153 | + | 230 | + | | |
| 77 | ++++ | 154 | +++++ | 231 | ++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 15:

In a 10 mL reaction bottle, 306 imine reductase enzyme solutions (a mass ratio of acetone to imine reductase in the enzyme solution was 1:8) shown in Table 1 were respectively added, then 4.48 mg of GDH, 21.6 mg of Glu, 1.33 mg of NAD⁺, 1.53 mg of NADP⁺, 4.48 mg (20 mM) of trifluoroacetone 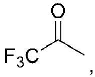 and 12 mg (120 mM) of hydrazine hydrate were added. The total volume was adjusted to 2 mL with 100 mM Tris-HCI buffer (pH 9.0), and the reaction was performed for 20 h at 30 °C through shaking. 500 µL of the system was taken, the reaction was terminated with 100 µL of a 0.1 M sodium hydroxide solution, centrifugation was performed for 10 min at 12000 rpm, extraction was performed using ethyl acetate, and then GC or LC-MS detection was performed to determine a conversion rate, as shown in Table 16 below.

**Table 16:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | + | 233 | + |
| 3 | + | 80 | + | 157 | + | 234 | + |
| 4 | + | 81 | + | 158 | + | 235 | + |
| 5 | + | 82 | + | 159 | + | 236 | + |
| 6 | + | 83 | + | 160 | + | 237 | + |
| 7 | + | 84 | + | 161 | + | 238 | + |
| 8 | + | 85 | + | 162 | + | 239 | + |
| 9 | +++ | 86 | + | 163 | + | 240 | + |
| 10 | + | 87 | + | 164 | + | 241 | ++ |
| 11 | + | 88 | + | 165 | + | 242 | + |
| 12 | + | 89 | + | 166 | + | 243 | + |
| 13 | + | 90 | + | 167 | + | 244 | + |
| 14 | + | 91 | + | 168 | + | 245 | + |
| 15 | +++ | 92 | + | 169 | + | 246 | + |
| 16 | + | 93 | + | 170 | + | 247 | + |
| 17 | + | 94 | + | 171 | + | 248 | + |
| 18 | +++ | 95 | + | 172 | + | 249 | + |
| 19 | + | 96 | + | 173 | + | 250 | + |
| 20 | +++ | 97 | + | 174 | + | 251 | + |
| 21 | + | 98 | + | 175 | + | 252 | +++ |
| 22 | ++++ | 99 | + | 176 | + | 253 | + |
| 23 | + | 100 | + | 177 | + | 254 | + |
| 24 | + | 101 | + | 178 | + | 255 | + |
| 25 | + | 102 | + | 179 | + | 256 | + |
| 26 | + | 103 | + | 180 | + | 257 | + |
| 27 | +++ | 104 | + | 181 | + | 258 | ++ |
| 28 | + | 105 | + | 182 | ++ | 259 | +++ |
| 29 | + | 106 | + | 183 | + | 260 | + |
| 30 | + | 107 | + | 184 | + | 261 | + |
| 31 | +++ | 108 | + | 185 | + | 262 | + |
| 32 | + | 109 | + | 186 | + | 263 | + |
| 33 | + | 110 | + | 187 | + | 264 | + |
| 34 | + | 111 | + | 188 | + | 265 | + |
| 35 | + | 112 | +++ | 189 | + | 266 | + |
| 36 | + | 113 | + | 190 | + | 267 | + |
| 37 | ++ | 114 | + | 191 | + | 268 | + |
| 38 | + | 115 | + | 192 | + | 269 | + |
| 39 | + | 116 | + | 193 | +++ | 270 | ++ |
| 40 | + | 117 | + | 194 | + | 271 | + |
| 41 | + | 118 | + | 195 | + | 272 | + |
| 42 | + | 119 | + | 196 | + | 273 | + |
| 43 | + | 120 | + | 197 | + | 274 | + |
| 44 | ++ | 121 | +++ | 198 | + | 275 | + |
| 45 | + | 122 | + | 199 | + | 276 | + |
| 46 | + | 123 | + | 200 | + | 277 | + |
| 47 | + | 124 | + | 201 | + | 278 | + |
| 48 | + | 125 | + | 202 | + | 279 | +++ |
| 49 | + | 126 | + | 203 | + | 280 | + |
| 50 | + | 127 | + | 204 | + | 281 | + |
| 51 | +++ | 128 | + | 205 | + | 282 | + |
| 52 | + | 129 | + | 206 | ++ | 283 | + |
| 53 | + | 130 | + | 207 | + | 284 | + |
| 54 | + | 131 | ++ | 208 | + | 285 | + |
| 55 | + | 132 | + | 209 | ++ | 286 | ++ |
| 56 | + | 133 | + | 210 | + | 287 | + |
| 57 | + | 134 | + | 211 | + | 288 | ++ |
| 58 | + | 135 | + | 212 | + | 289 | + |
| 59 | + | 136 | + | 213 | + | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | + | 140 | + | 217 | + | 294 | + |
| 64 | + | 141 | + | 218 | + | 295 | + |
| 65 | + | 142 | + | 219 | + | 296 | + |
| 66 | +++ | 143 | + | 220 | +++ | 297 | ++ |
| 67 | + | 144 | + | 221 | + | 298 | + |
| 68 | + | 145 | + | 222 | + | 299 | + |
| 69 | + | 146 | + | 223 | + | 300 | + |
| 70 | + | 147 | + | 224 | + | 301 | +++ |
| 71 | + | 148 | + | 225 | + | 302 | + |
| 72 | + | 149 | + | 226 | + | 303 | + |
| 73 | + | 150 | + | 227 | + | 304 | + |
| 74 | + | 151 | + | 228 | ++ | 305 | + |
| 75 | + | 152 | + | 229 | + | 306 | + |
| 76 | ++ | 153 | + | 230 | + | | |
| 77 | + | 154 | +++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 16:

In a 10 mL reaction bottle, 306 imine reductase enzyme solutions (a mass ratio of substrate ketone to imine reductase in the enzyme solution was 1:6) shown in Table 1 were respectively added, then 3.92 mg of GDH, 21.6 mg of Glu, 1.33 mg of NAD⁺, 1.53 mg of NADP⁺, 3.92 mg (20 mM) of cyclohexanone 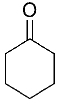 (dissolved in 10 µL of DMSO), and 35.2 mg (200 mM) of tert-butyl-hydrazine 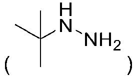 were added. The total volume was adjusted to 2 mL with 100 mM Tris-HCI buffer (pH 9.0), and the reaction was performed for 16 h at 37 °C through shaking. 500 µL of the system was taken, the reaction was terminated with 100 µL of a 0.1 M sodium hydroxide solution, full shaking and well mixing were performed, then centrifugation was performed for 10 min at 12000 rpm, and then GC or LC-MS detection was performed to determine a conversion rate, as shown in Table 17 below.

**Table 17:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | ++ | 79 | + | 156 | + | 233 | + |
| 3 | + | 80 | + | 157 | + | 234 | + |
| 4 | +++ | 81 | +++ | 158 | +++ | 235 | + |
| 5 | + | 82 | + | 159 | + | 236 | +++ |
| 6 | + | 83 | + | 160 | + | 237 | +++ |
| 7 | + | 84 | + | 161 | + | 238 | + |
| 8 | ++++ | 85 | + | 162 | + | 239 | + |
| 9 | + | 86 | + | 163 | ++ | 240 | + |
| 10 | +++ | 87 | + | 164 | ++ | 241 | + |
| 11 | + | 88 | + | 165 | + | 242 | + |
| 12 | + | 89 | + | 166 | + | 243 | +++ |
| 13 | + | 90 | + | 167 | +++ | 244 | + |
| 14 | + | 91 | + | 168 | + | 245 | + |
| 15 | ++++ | 92 | + | 169 | + | 246 | + |
| 16 | + | 93 | + | 170 | + | 247 | + |
| 17 | + | 94 | + | 171 | + | 248 | + |
| 18 | + | 95 | + | 172 | +++ | 249 | +++ |
| 19 | + | 96 | + | 173 | +++ | 250 | + |
| 20 | + | 97 | + | 174 | + | 251 | + |
| 21 | ++++ | 98 | + | 175 | ++ | 252 | + |
| 22 | ++ | 99 | + | 176 | + | 253 | + |
| 23 | + | 100 | +++ | 177 | +++ | 254 | + |
| 24 | + | 101 | +++++ | 178 | + | 255 | +++ |
| 25 | +++ | 102 | + | 179 | + | 256 | + |
| 26 | ++++ | 103 | + | 180 | + | 257 | +++ |
| 27 | + | 104 | + | 181 | + | 258 | + |
| 28 | + | 105 | + | 182 | + | 259 | + |
| 29 | + | 106 | +++++ | 183 | + | 260 | ++ |
| 30 | + | 107 | + | 184 | +++++ | 261 | + |
| 31 | +++++ | 108 | +++ | 185 | + | 262 | +++ |
| 32 | + | 109 | + | 186 | + | 263 | + |
| 33 | + | 110 | + | 187 | ++ | 264 | ++ |
| 34 | + | 111 | ++ | 188 | + | 265 | + |
| 35 | + | 112 | ++ | 189 | + | 266 | + |
| 36 | + | 113 | + | 190 | + | 267 | + |
| 37 | + | 114 | + | 191 | +++ | 268 | + |
| 38 | +++ | 115 | ++++ | 192 | +++ | 269 | +++ |
| 39 | + | 116 | + | 193 | + | 270 | + |
| 40 | + | 117 | + | 194 | + | 271 | + |
| 41 | ++++ | 118 | +++ | 195 | +++ | 272 | ++ |
| 42 | + | 119 | + | 196 | + | 273 | ++ |
| 43 | + | 120 | + | 197 | + | 274 | + |
| 44 | ++ | 121 | +++ | 198 | ++ | 275 | + |
| 45 | + | 122 | +++ | 199 | + | 276 | + |
| 46 | + | 123 | + | 200 | + | 277 | + |
| 47 | + | 124 | + | 201 | + | 278 | + |
| 48 | + | 125 | + | 202 | +++ | 279 | + |
| 49 | + | 126 | +++ | 203 | + | 280 | +++ |
| 50 | + | 127 | + | 204 | + | 281 | +++ |
| 51 | + | 128 | + | 205 | + | 282 | + |
| 52 | +++++ | 129 | +++ | 206 | +++ | 283 | + |
| 53 | + | 130 | + | 207 | + | 284 | + |
| 54 | + | 131 | + | 208 | ++ | 285 | +++ |
| 55 | + | 132 | + | 209 | + | 286 | ++ |
| 56 | + | 133 | + | 210 | + | 287 | + |
| 57 | + | 134 | +++++ | 211 | + | 288 | ++ |
| 58 | + | 135 | + | 212 | +++ | 289 | + |
| 59 | + | 136 | + | 213 | + | 290 | + |
| 60 | + | 137 | +++ | 214 | + | 291 | +++ |
| 61 | + | 138 | ++ | 215 | +++ | 292 | + |
| 62 | + | 139 | ++ | 216 | + | 293 | +++++ |
| 63 | + | 140 | + | 217 | +++ | 294 | + |
| 64 | + | 141 | + | 218 | + | 295 | + |
| 65 | + | 142 | + | 219 | + | 296 | + |
| 66 | + | 143 | ++++ | 220 | + | 297 | + |
| 67 | + | 144 | + | 221 | +++ | 298 | +++ |
| 68 | + | 145 | +++ | 222 | + | 299 | +++ |
| 69 | + | 146 | ++ | 223 | + | 300 | + |
| 70 | + | 147 | + | 224 | +++ | 301 | ++ |
| 71 | + | 148 | +++ | 225 | + | 302 | + |
| 72 | +++ | 149 | + | 226 | + | 303 | + |
| 73 | + | 150 | + | 227 | + | 304 | +++ |
| 74 | + | 151 | ++ | 228 | + | 305 | ++ |
| 75 | + | 152 | ++ | 229 | + | 306 | + |
| 76 | +++++ | 153 | + | 230 | +++ | | |
| 77 | + | 154 | +++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 17:

In a 10 mL reaction bottle, 306 imine reductase enzyme solutions (a mass ratio of substrate ketone to imine reductase in the enzyme solution was 1:10) shown in Table 1 were respectively added, then 7.84 mg of GDH, 21.6 mg of Glu, 1.33 mg of NAD⁺, 1.53 mg of NADP⁺, 7.84 mg (40 mM) of cyclohexanone 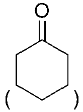 (dissolved in 10 uL of DMSO), and 43.2 mg (200 mM) of phenylhydrazine 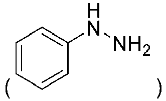 were added. The total volume was adjusted to 2 mL with 100 mM Tris-HCI buffer (pH 8.5), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 18 below.

**Table 18:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | + | 233 | + |
| 3 | + | 80 | + | 157 | + | 234 | + |
| 4 | + | 81 | + | 158 | + | 235 | + |
| 5 | + | 82 | + | 159 | + | 236 | + |
| 6 | + | 83 | + | 160 | + | 237 | + |
| 7 | + | 84 | + | 161 | + | 238 | + |
| 8 | + | 85 | + | 162 | + | 239 | ++ |
| 9 | + | 86 | + | 163 | + | 240 | + |
| 10 | ++ | 87 | ++ | 164 | + | 241 | + |
| 11 | + | 88 | + | 165 | ++++ | 242 | + |
| 12 | + | 89 | + | 166 | + | 243 | + |
| 13 | + | 90 | + | 167 | + | 244 | + |
| 14 | + | 91 | + | 168 | + | 245 | + |
| 15 | + | 92 | + | 169 | + | 246 | + |
| 16 | ++ | 93 | + | 170 | + | 247 | + |
| 17 | + | 94 | + | 171 | + | 248 | + |
| 18 | + | 95 | + | 172 | + | 249 | ++++ |
| 19 | + | 96 | + | 173 | + | 250 | + |
| 20 | + | 97 | + | 174 | + | 251 | ++ |
| 21 | + | 98 | + | 175 | + | 252 | + |
| 22 | ++ | 99 | + | 176 | + | 253 | + |
| 23 | + | 100 | ++ | 177 | + | 254 | + |
| 24 | + | 101 | + | 178 | + | 255 | + |
| 25 | +++ | 102 | + | 179 | + | 256 | + |
| 26 | +++ | 103 | + | 180 | + | 257 | + |
| 27 | + | 104 | + | 181 | + | 258 | + |
| 28 | + | 105 | + | 182 | + | 259 | + |
| 29 | + | 106 | + | 183 | + | 260 | + |
| 30 | + | 107 | ++++ | 184 | + | 261 | + |
| 31 | +++ | 108 | + | 185 | + | 262 | + |
| 32 | + | 109 | + | 186 | ++ | 263 | + |
| 33 | + | 110 | + | 187 | + | 264 | + |
| 34 | + | 111 | + | 188 | + | 265 | + |
| 35 | + | 112 | + | 189 | + | 266 | + |
| 36 | + | 113 | + | 190 | + | 267 | + |
| 37 | + | 114 | + | 191 | + | 268 | + |
| 38 | + | 115 | ++ | 192 | ++ | 269 | + |
| 39 | + | 116 | + | 193 | + | 270 | + |
| 40 | + | 117 | + | 194 | + | 271 | + |
| 41 | ++ | 118 | + | 195 | + | 272 | + |
| 42 | + | 119 | + | 196 | + | 273 | + |
| 43 | + | 120 | + | 197 | + | 274 | + |
| 44 | + | 121 | + | 198 | + | 275 | + |
| 45 | + | 122 | + | 199 | + | 276 | + |
| 46 | + | 123 | + | 200 | + | 277 | + |
| 47 | + | 124 | + | 201 | + | 278 | ++ |
| 48 | + | 125 | + | 202 | + | 279 | + |
| 49 | + | 126 | + | 203 | ++ | 280 | + |
| 50 | + | 127 | + | 204 | + | 281 | + |
| 51 | + | 128 | + | 205 | + | 282 | + |
| 52 | + | 129 | ++ | 206 | + | 283 | + |
| 53 | + | 130 | + | 207 | + | 284 | + |
| 54 | + | 131 | + | 208 | + | 285 | + |
| 55 | + | 132 | + | 209 | + | 286 | + |
| 56 | ++ | 133 | + | 210 | + | 287 | + |
| 57 | + | 134 | + | 211 | + | 288 | + |
| 58 | + | 135 | + | 212 | ++ | 289 | + |
| 59 | + | 136 | + | 213 | + | 290 | + |
| 60 | + | 137 | + | 214 | + | 291 | ++ |
| 61 | + | 138 | + | 215 | + | 292 | + |
| 62 | + | 139 | + | 216 | + | 293 | + |
| 63 | + | 140 | + | 217 | + | 294 | + |
| 64 | + | 141 | + | 218 | + | 295 | + |
| 65 | + | 142 | + | 219 | + | 296 | + |
| 66 | + | 143 | + | 220 | + | 297 | ++++ |
| 67 | + | 144 | + | 221 | + | 298 | + |
| 68 | + | 145 | + | 222 | + | 299 | + |
| 69 | + | 146 | + | 223 | + | 300 | + |
| 70 | + | 147 | + | 224 | + | 301 | ++ |
| 71 | + | 148 | + | 225 | + | 302 | + |
| 72 | + | 149 | + | 226 | + | 303 | + |
| 73 | + | 150 | + | 227 | + | 304 | + |
| 74 | + | 151 | + | 228 | + | 305 | + |
| 75 | + | 152 | + | 229 | + | 306 | + |
| 76 | ++++ | 153 | + | 230 | + | | |
| 77 | + | 154 | + | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 18:

In a 10 mL reaction bottle, 306 imine reductase enzyme solutions (a mass ratio of substrate ketone to imine reductase in the enzyme solution was 1:5) shown in Table 1 were respectively added, then 15.68 mg of GDH, 21.6 mg of Glu, 1.33 mg of NAD⁺, 1.53 mg of NADP⁺, 15.68 mg (80 mM) of cyclohexanone 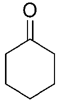 (dissolved in 10 µL of DMSO), and 30.4 mg (200 mM) of 2-hydroxyethylhydrazine 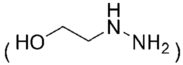 were added. The total volume was adjusted to 2 mL with 100 mM Tris-HCI buffer (pH 9.0), and the reaction was performed for 16 h at 37 °C through shaking. 500 µL of the system was taken, the reaction was terminated with 100 µL of a 0.1 M sodium hydroxide solution, full shaking and well mixing were performed, then centrifugation was performed for 10 min at 12000 rpm, and then GC or LC-MS detection was performed to determine a conversion rate, as shown in Table 19 below.

**Table 19:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | ++++ | 232 | ++ |
| 2 | ++++ | 79 | + | 156 | + | 233 | + |
| 3 | + | 80 | + | 157 | +++ | 234 | +++ |
| 4 | ++++ | 81 | ++++ | 158 | +++ | 235 | +++ |
| 5 | + | 82 | + | 159 | ++ | 236 | ++ |
| 6 | + | 83 | + | 160 | + | 237 | + |
| 7 | + | 84 | + | 161 | + | 238 | + |
| 8 | ++++ | 85 | + | 162 | + | 239 | +++++ |
| 9 | + | 86 | +++ | 163 | +++++ | 240 | + |
| 10 | ++++ | 87 | + | 164 | ++ | 241 | +++ |
| 11 | + | 88 | + | 165 | ++ | 242 | ++ |
| 12 | +++ | 89 | ++++ | 166 | + | 243 | + |
| 13 | +++ | 90 | ++++ | 167 | + | 244 | +++ |
| 14 | + | 91 | + | 168 | +++++ | 245 | ++ |
| 15 | ++++ | 92 | + | 169 | + | 246 | + |
| 16 | ++++ | 93 | + | 170 | ++++ | 247 | + |
| 17 | + | 94 | + | 171 | ++ | 248 | + |
| 18 | +++ | 95 | + | 172 | + | 249 | +++ |
| 19 | + | 96 | ++++ | 173 | + | 250 | +++ |
| 20 | ++++ | 97 | + | 174 | +++ | 251 | ++ |
| 21 | ++++ | 98 | + | 175 | +++ | 252 | + |
| 22 | ++++ | 99 | ++++ | 176 | + | 253 | ++ |
| 23 | + | 100 | + | 177 | ++ | 254 | + |
| 24 | ++++ | 101 | + | 178 | ++ | 255 | +++++ |
| 25 | +++++ | 102 | + | 179 | + | 256 | +++ |
| 26 | +++++ | 103 | + | 180 | +++++ | 257 | + |
| 27 | +++ | 104 | + | 181 | + | 258 | + |
| 28 | +++ | 105 | ++++ | 182 | +++++ | 259 | +++ |
| 29 | +++ | 106 | + | 183 | + | 260 | + |
| 30 | + | 107 | ++++ | 184 | +++ | 261 | + |
| 31 | +++++ | 108 | ++++ | 185 | + | 262 | + |
| 32 | + | 109 | + | 186 | + | 263 | +++ |
| 33 | + | 110 | + | 187 | ++++ | 264 | +++ |
| 34 | +++ | 111 | ++++ | 188 | + | 265 | + |
| 35 | + | 112 | + | 189 | + | 266 | + |
| 36 | + | 113 | +++ | 190 | +++ | 267 | + |
| 37 | ++++ | 114 | +++ | 191 | +++ | 268 | + |
| 38 | ++ | 115 | + | 192 | + | 269 | ++++ |
| 39 | + | 116 | ++++ | 193 | + | 270 | + |
| 40 | +++ | 117 | + | 194 | ++++ | 271 | + |
| 41 | +++++ | 118 | +++ | 195 | ++++ | 272 | +++++ |
| 42 | +++ | 119 | +++ | 196 | ++ | 273 | +++ |
| 43 | +++ | 120 | + | 197 | + | 274 | ++ |
| 44 | ++++ | 121 | ++++ | 198 | +++ | 275 | + |
| 45 | + | 122 | ++ | 199 | + | 276 | + |
| 46 | + | 123 | + | 200 | ++ | 277 | +++ |
| 47 | +++ | 124 | +++++ | 201 | + | 278 | +++ |
| 48 | + | 125 | + | 202 | + | 279 | + |
| 49 | + | 126 | + | 203 | +++++ | 280 | ++++ |
| 50 | +++ | 127 | + | 204 | +++ | 281 | + |
| 51 | + | 128 | ++++ | 205 | +++ | 282 | + |
| 52 | ++ | 129 | + | 206 | ++ | 283 | + |
| 53 | ++ | 130 | ++++ | 207 | +++ | 284 | + |
| 54 | + | 131 | + | 208 | ++ | 285 | + |
| 55 | + | 132 | +++ | 209 | + | 286 | +++ |
| 56 | ++ | 133 | +++ | 210 | + | 287 | + |
| 57 | + | 134 | +++ | 211 | ++++ | 288 | + |
| 58 | + | 135 | ++ | 212 | ++++ | 289 | ++++ |
| 59 | +++ | 136 | ++ | 213 | + | 290 | +++ |
| 60 | + | 137 | + | 214 | + | 291 | + |
| 61 | + | 138 | + | 215 | + | 292 | + |
| 62 | + | 139 | + | 216 | +++ | 293 | +++ |
| 63 | + | 140 | + | 217 | ++ | 294 | +++ |
| 64 | + | 141 | ++++ | 218 | + | 295 | + |
| 65 | +++ | 142 | + | 219 | +++ | 296 | + |
| 66 | + | 143 | + | 220 | ++ | 297 | + |
| 67 | + | 144 | ++++ | 221 | ++ | 298 | + |
| 68 | + | 145 | + | 222 | + | 299 | + |
| 69 | + | 146 | + | 223 | +++++ | 300 | ++++ |
| 70 | + | 147 | +++ | 224 | + | 301 | +++ |
| 71 | +++ | 148 | +++ | 225 | +++ | 302 | ++++ |
| 72 | ++++ | 149 | + | 226 | ++++ | 303 | + |
| 73 | + | 150 | +++++ | 227 | + | 304 | + |
| 74 | + | 151 | ++ | 228 | ++ | 305 | +++ |
| 75 | + | 152 | + | 229 | + | 306 | ++ |
| 76 | +++++ | 153 | + | 230 | +++ | | |
| 77 | + | 154 | ++++ | 231 | +++ | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 19:

In a 10 mL reaction bottle, 306 imine reductase enzyme solutions (a mass ratio of substrate ketone to imine reductase in the enzyme solution was 1:10) shown in Table 1 were respectively added, then 3.92 mg of GDH, 21.6 mg of Glu, 1.33 mg of NAD⁺, 1.53 mg of NADP⁺, 3.92 mg (20 mM) of cyclohexanone 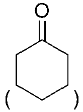 (dissolved in 10 µL of DMSO), and 12.3 mg (200 mM) of benzylhydrazine (NH₂NH₂Bn) were added. The total volume was adjusted to 2 mL with 100 mM Tris-HCI buffer (pH 8.5), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 20 below.

**Table 20:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | ++ | 79 | + | 156 | + | 233 | + |
| 3 | + | 80 | + | 157 | + | 234 | + |
| 4 | +++ | 81 | +++ | 158 | +++ | 235 | + |
| 5 | + | 82 | + | 159 | + | 236 | + |
| 6 | + | 83 | + | 160 | + | 237 | ++ |
| 7 | + | 84 | + | 161 | + | 238 | + |
| 8 | ++++ | 85 | + | 162 | + | 239 | + |
| 9 | + | 86 | + | 163 | ++ | 240 | + |
| 10 | +++ | 87 | + | 164 | ++ | 241 | + |
| 11 | + | 88 | + | 165 | + | 242 | + |
| 12 | + | 89 | + | 166 | + | 243 | +++ |
| 13 | + | 90 | + | 167 | +++ | 244 | + |
| 14 | + | 91 | + | 168 | + | 245 | + |
| 15 | ++++ | 92 | + | 169 | + | 246 | + |
| 16 | + | 93 | + | 170 | + | 247 | + |
| 17 | + | 94 | + | 171 | + | 248 | + |
| 18 | + | 95 | + | 172 | +++ | 249 | +++ |
| 19 | + | 96 | + | 173 | +++ | 250 | + |
| 20 | + | 97 | + | 174 | + | 251 | + |
| 21 | ++++ | 98 | + | 175 | ++ | 252 | + |
| 22 | ++ | 99 | + | 176 | + | 253 | + |
| 23 | + | 100 | +++ | 177 | +++ | 254 | + |
| 24 | + | 101 | ++++ | 178 | + | 255 | +++ |
| 25 | ++++ | 102 | + | 179 | + | 256 | + |
| 26 | +++ | 103 | + | 180 | + | 257 | +++ |
| 27 | + | 104 | + | 181 | + | 258 | + |
| 28 | + | 105 | + | 182 | + | 259 | + |
| 29 | + | 106 | ++++ | 183 | + | 260 | ++ |
| 30 | + | 107 | + | 184 | +++++ | 261 | + |
| 31 | ++++ | 108 | +++ | 185 | + | 262 | +++ |
| 32 | + | 109 | + | 186 | + | 263 | + |
| 33 | + | 110 | + | 187 | ++ | 264 | ++ |
| 34 | + | 111 | ++ | 188 | + | 265 | + |
| 35 | + | 112 | ++ | 189 | + | 266 | + |
| 36 | + | 113 | + | 190 | + | 267 | + |
| 37 | + | 114 | + | 191 | +++ | 268 | + |
| 38 | +++ | 115 | ++++ | 192 | + | 269 | +++ |
| 39 | + | 116 | + | 193 | + | 270 | + |
| 40 | + | 117 | + | 194 | + | 271 | + |
| 41 | ++++ | 118 | +++ | 195 | +++ | 272 | ++ |
| 42 | + | 119 | + | 196 | + | 273 | ++ |
| 43 | + | 120 | + | 197 | + | 274 | + |
| 44 | ++ | 121 | + | 198 | ++ | 275 | + |
| 45 | + | 122 | +++ | 199 | + | 276 | + |
| 46 | + | 123 | + | 200 | + | 277 | + |
| 47 | + | 124 | + | 201 | + | 278 | + |
| 48 | + | 125 | + | 202 | +++ | 279 | + |
| 49 | + | 126 | +++ | 203 | + | 280 | +++ |
| 50 | + | 127 | + | 204 | + | 281 | +++ |
| 51 | + | 128 | + | 205 | + | 282 | + |
| 52 | ++ | 129 | +++ | 206 | +++ | 283 | + |
| 53 | + | 130 | + | 207 | + | 284 | + |
| 54 | + | 131 | + | 208 | ++ | 285 | +++ |
| 55 | + | 132 | + | 209 | + | 286 | ++ |
| 56 | + | 133 | + | 210 | + | 287 | + |
| 57 | + | 134 | +++++ | 211 | + | 288 | ++ |
| 58 | + | 135 | + | 212 | +++ | 289 | + |
| 59 | + | 136 | + | 213 | + | 290 | + |
| 60 | + | 137 | +++ | 214 | + | 291 | +++ |
| 61 | + | 138 | ++ | 215 | +++ | 292 | + |
| 62 | + | 139 | ++ | 216 | + | 293 | +++++ |
| 63 | + | 140 | + | 217 | +++ | 294 | + |
| 64 | + | 141 | + | 218 | + | 295 | + |
| 65 | + | 142 | + | 219 | + | 296 | + |
| 66 | + | 143 | ++++ | 220 | + | 297 | + |
| 67 | + | 144 | + | 221 | +++ | 298 | +++ |
| 68 | + | 145 | +++ | 222 | + | 299 | +++ |
| 69 | + | 146 | ++ | 223 | + | 300 | + |
| 70 | + | 147 | + | 224 | +++ | 301 | ++ |
| 71 | + | 148 | + | 225 | + | 302 | ++++ |
| 72 | +++ | 149 | + | 226 | + | 303 | + |
| 73 | + | 150 | + | 227 | + | 304 | ++ |
| 74 | + | 151 | ++ | 228 | + | 305 | ++ |
| 75 | + | 152 | ++ | 229 | + | 306 | + |
| 76 | +++++ | 153 | + | 230 | +++ | | |
| 77 | + | 154 | +++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 20:

In a 10 mL reaction bottle, 306 imine reductase enzyme solutions (a mass ratio of substrate ketone to imine reductase in the enzyme solution was 1:10) shown in Table 1 were respectively added, then 3.92 mg of GDH, 21.6 mg of Glu, 1.33 mg of NAD⁺, 1.53 mg of NADP⁺, 3.92 mg (20 mM) of cyclohexanone 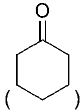 (dissolved in 10 µL of DMSO), and 54.5 mg (200 mM) of benzoyl hydrazine 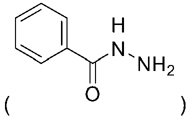 were added. The total volume was adjusted to 2 mL with 100 mM Tris-HCI buffer (pH 9.0), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and HPLC or LC-MS detection was performed to determine a conversion rate, as shown in Table 21 below.

**Table 21:**

| Enzyme number | Conversion rate | Enzyme number | Conversion rate | Enzyme number | Conversion rate | Enzyme number | Conversion rate |
|---|---|---|---|---|---|---|---|
| 1 | + | 78 | + | 155 | + | 232 | + |
| 2 | + | 79 | + | 156 | + | 233 | + |
| 3 | + | 80 | + | 157 | + | 234 | + |
| 4 | +++ | 81 | +++ | 158 | +++ | 235 | + |
| 5 | ++++ | 82 | + | 159 | + | 236 | + |
| 6 | + | 83 | + | 160 | + | 237 | ++ |
| 7 | + | 84 | + | 161 | + | 238 | + |
| 8 | ++++ | 85 | + | 162 | + | 239 | + |
| 9 | + | 86 | ++ | 163 | ++ | 240 | + |
| 10 | +++ | 87 | + | 164 | ++ | 241 | + |
| 11 | + | 88 | + | 165 | + | 242 | + |
| 12 | + | 89 | + | 166 | + | 243 | +++ |
| 13 | + | 90 | ++ | 167 | +++ | 244 | + |
| 14 | + | 91 | + | 168 | + | 245 | + |
| 15 | ++++ | 92 | + | 169 | + | 246 | + |
| 16 | + | 93 | ++ | 170 | + | 247 | + |
| 17 | + | 94 | + | 171 | + | 248 | + |
| 18 | +++ | 95 | + | 172 | +++ | 249 | +++ |
| 19 | + | 96 | ++ | 173 | +++ | 250 | + |
| 20 | ++++ | 97 | + | 174 | + | 251 | + |
| 21 | ++++ | 98 | + | 175 | ++ | 252 | + |
| 22 | ++ | 99 | + | 176 | + | 253 | + |
| 23 | + | 100 | +++ | 177 | +++ | 254 | + |
| 24 | + | 101 | ++++ | 178 | + | 255 | +++ |
| 25 | ++++ | 102 | + | 179 | + | 256 | + |
| 26 | +++ | 103 | + | 180 | + | 257 | +++ |
| 27 | + | 104 | + | 181 | + | 258 | + |
| 28 | + | 105 | + | 182 | + | 259 | + |
| 29 | + | 106 | ++++ | 183 | + | 260 | ++ |
| 30 | + | 107 | + | 184 | +++++ | 261 | + |
| 31 | ++++ | 108 | +++ | 185 | + | 262 | +++ |
| 32 | + | 109 | + | 186 | + | 263 | + |
| 33 | + | 110 | + | 187 | ++ | 264 | ++ |
| 34 | + | 111 | ++ | 188 | + | 265 | + |
| 35 | + | 112 | ++ | 189 | + | 266 | + |
| 36 | + | 113 | + | 190 | + | 267 | + |
| 37 | ++++ | 114 | + | 191 | +++ | 268 | + |
| 38 | +++ | 115 | ++++ | 192 | + | 269 | +++ |
| 39 | + | 116 | + | 193 | + | 270 | + |
| 40 | + | 117 | + | 194 | + | 271 | + |
| 41 | ++++ | 118 | +++ | 195 | +++ | 272 | ++ |
| 42 | + | 119 | + | 196 | + | 273 | ++ |
| 43 | + | 120 | + | 197 | + | 274 | + |
| 44 | ++ | 121 | + | 198 | ++ | 275 | + |
| 45 | + | 122 | +++ | 199 | + | 276 | + |
| 46 | + | 123 | + | 200 | + | 277 | + |
| 47 | + | 124 | + | 201 | + | 278 | + |
| 48 | + | 125 | + | 202 | +++ | 279 | + |
| 49 | + | 126 | +++ | 203 | + | 280 | +++ |
| 50 | + | 127 | + | 204 | + | 281 | +++ |
| 51 | + | 128 | + | 205 | + | 282 | + |
| 52 | ++ | 129 | +++ | 206 | +++ | 283 | + |
| 53 | + | 130 | + | 207 | + | 284 | + |
| 54 | + | 131 | + | 208 | ++ | 285 | +++ |
| 55 | + | 132 | + | 209 | + | 286 | ++ |
| 56 | + | 133 | + | 210 | + | 287 | + |
| 57 | + | 134 | +++++ | 211 | + | 288 | ++ |
| 58 | +++ | 135 | + | 212 | +++ | 289 | + |
| 59 | + | 136 | + | 213 | + | 290 | + |
| 60 | + | 137 | +++ | 214 | + | 291 | +++ |
| 61 | +++ | 138 | ++ | 215 | +++ | 292 | + |
| 62 | + | 139 | ++ | 216 | + | 293 | +++++ |
| 63 | + | 140 | + | 217 | +++ | 294 | + |
| 64 | + | 141 | + | 218 | + | 295 | + |
| 65 | + | 142 | + | 219 | + | 296 | + |
| 66 | + | 143 | ++++ | 220 | + | 297 | + |
| 67 | + | 144 | + | 221 | +++ | 298 | +++ |
| 68 | + | 145 | ++ | 222 | + | 299 | +++ |
| 69 | + | 146 | ++ | 223 | + | 300 | + |
| 70 | + | 147 | + | 224 | +++ | 301 | ++ |
| 71 | + | 148 | + | 225 | + | 302 | ++++ |
| 72 | +++ | 149 | + | 226 | + | 303 | + |
| 73 | + | 150 | + | 227 | + | 304 | ++ |
| 74 | + | 151 | ++ | 228 | + | 305 | + |
| 75 | + | 152 | + | 229 | + | 306 | + |
| 76 | ++++ | 153 | + | 230 | +++ | | |
| 77 | + | 154 | ++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 21:

In a 10 mL reaction bottle, 306 imine reductase enzyme solutions (a mass ratio of substrate ketone to imine reductase in the enzyme solution was 1:10) shown in Table 1 were respectively added, then 3.92 mg of GDH, 21.6 mg of Glu, 1.33 mg of NAD⁺, 1.53 mg of NADP⁺, 3.92 mg (20 mM) of cyclohexanone 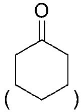 (dissolved in 10 µL of DMSO), and 29.6 mg (200 mM) of acetylhydrazine 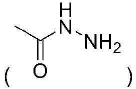 were added. The total volume was adjusted to 2 mL with 100 mM Tris-HCI buffer (pH 9.0), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and LC-MS detection was performed; or 500 µL of the system was taken, the reaction was terminated with 100 µL of a 0.1 M sodium hydroxide solution, centrifugation was performed for 10 min at 12000 rpm, extraction was performed using ethyl acetate, and then GC detection was performed to determine a conversion rate, as shown in Table 22.

**Table 22:**

| Enzyme number | Conversion rate | Enzyme number | Conversion rate | Enzyme number | Conversion rate | Enzyme number | Conversion rate |
|---|---|---|---|---|---|---|---|
| 1 | ++ | 78 | + | 155 | + | 232 | + |
| 2 | + | 79 | + | 156 | + | 233 | + |
| 3 | + | 80 | + | 157 | + | 234 | + |
| 4 | ++ | 81 | +++ | 158 | +++ | 235 | + |
| 5 | +++ | 82 | + | 159 | + | 236 | +++ |
| 6 | + | 83 | + | 160 | + | 237 | + |
| 7 | + | 84 | + | 161 | + | 238 | + |
| 8 | ++ | 85 | + | 162 | + | 239 | + |
| 9 | + | 86 | ++++ | 163 | ++ | 240 | + |
| 10 | +++ | 87 | + | 164 | ++ | 241 | + |
| 11 | + | 88 | + | 165 | + | 242 | + |
| 12 | + | 89 | + | 166 | + | 243 | +++ |
| 13 | + | 90 | + | 167 | +++ | 244 | + |
| 14 | + | 91 | + | 168 | + | 245 | + |
| 15 | ++++ | 92 | + | 169 | + | 246 | + |
| 16 | + | 93 | + | 170 | + | 247 | + |
| 17 | + | 94 | + | 171 | + | 248 | + |
| 18 | + | 95 | + | 172 | ++ | 249 | +++ |
| 19 | + | 96 | + | 173 | ++ | 250 | + |
| 20 | +++ | 97 | + | 174 | + | 251 | + |
| 21 | + | 98 | + | 175 | ++ | 252 | + |
| 22 | ++ | 99 | + | 176 | + | 253 | + |
| 23 | + | 100 | +++ | 177 | +++ | 254 | + |
| 24 | + | 101 | ++ | 178 | + | 255 | +++ |
| 25 | +++ | 102 | + | 179 | + | 256 | + |
| 26 | + | 103 | + | 180 | + | 257 | +++ |
| 27 | ++++ | 104 | + | 181 | + | 258 | + |
| 28 | + | 105 | + | 182 | + | 259 | + |
| 29 | + | 106 | + | 183 | + | 260 | ++ |
| 30 | + | 107 | + | 184 | ++ | 261 | + |
| 31 | + | 108 | +++ | 185 | + | 262 | ++ |
| 32 | + | 109 | + | 186 | + | 263 | + |
| 33 | + | 110 | +++ | 187 | ++ | 264 | ++ |
| 34 | + | 111 | ++ | 188 | + | 265 | + |
| 35 | + | 112 | ++ | 189 | + | 266 | + |
| 36 | + | 113 | + | 190 | + | 267 | + |
| 37 | + | 114 | + | 191 | +++ | 268 | + |
| 38 | +++ | 115 | ++++ | 192 | +++ | 269 | +++ |
| 39 | + | 116 | + | 193 | + | 270 | + |
| 40 | + | 117 | + | 194 | + | 271 | + |
| 41 | ++++ | 118 | +++ | 195 | +++ | 272 | ++ |
| 42 | + | 119 | + | 196 | + | 273 | ++ |
| 43 | + | 120 | + | 197 | + | 274 | + |
| 44 | ++ | 121 | +++ | 198 | ++ | 275 | + |
| 45 | + | 122 | +++ | 199 | + | 276 | + |
| 46 | + | 123 | + | 200 | + | 277 | + |
| 47 | + | 124 | + | 201 | + | 278 | + |
| 48 | + | 125 | + | 202 | +++ | 279 | + |
| 49 | + | 126 | +++ | 203 | + | 280 | ++ |
| 50 | + | 127 | + | 204 | + | 281 | ++ |
| 51 | + | 128 | + | 205 | + | 282 | + |
| 52 | ++ | 129 | +++ | 206 | +++ | 283 | + |
| 53 | + | 130 | + | 207 | + | 284 | + |
| 54 | + | 131 | + | 208 | ++ | 285 | +++ |
| 55 | + | 132 | + | 209 | + | 286 | ++ |
| 56 | + | 133 | + | 210 | + | 287 | + |
| 57 | + | 134 | +++ | 211 | + | 288 | ++ |
| 58 | +++ | 135 | + | 212 | +++ | 289 | + |
| 59 | ++++ | 136 | + | 213 | + | 290 | + |
| 60 | + | 137 | +++ | 214 | + | 291 | +++ |
| 61 | + | 138 | ++ | 215 | +++ | 292 | + |
| 62 | + | 139 | + | 216 | + | 293 | ++++ |
| 63 | + | 140 | + | 217 | +++ | 294 | + |
| 64 | + | 141 | + | 218 | + | 295 | + |
| 65 | + | 142 | + | 219 | + | 296 | + |
| 66 | + | 143 | ++++ | 220 | + | 297 | + |
| 67 | + | 144 | + | 221 | +++ | 298 | +++ |
| 68 | + | 145 | +++ | 222 | + | 299 | +++ |
| 69 | + | 146 | ++ | 223 | + | 300 | + |
| 70 | + | 147 | + | 224 | +++ | 301 | ++ |
| 71 | + | 148 | ++ | 225 | + | 302 | + |
| 72 | +++ | 149 | + | 226 | + | 303 | + |
| 73 | + | 150 | + | 227 | + | 304 | ++ |
| 74 | + | 151 | ++ | 228 | + | 305 | ++ |
| 75 | + | 152 | ++ | 229 | + | 306 | + |
| 76 | +++ | 153 | + | 230 | +++ | | |
| 77 | + | 154 | +++ | 231 | + | | |

In the table above, + indicated that the conversion rate was 0.01-1%, ++ indicated that the conversion rate was 1-10%, +++ indicated that the conversion rate was 10%-30%, ++++ indicated that the conversion rate was 30-50%, and +++++ indicated that the conversion rate was 50% or higher.

### Embodiment 22:

In a 10 mL reaction bottle, 306 imine reductase enzyme solutions (a mass ratio of substrate ketone to imine reductase in the enzyme solution was 1:10) shown in Table 1 were respectively added, then 3.92 mg of GDH, 21.6 mg of Glu, 1.33 mg of NAD⁺, 1.53 mg of NADP⁺, 1.96 mg (10 mM) of cyclohexanone 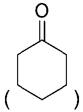 (dissolved in 20 pL of DMSO), and 50.5 mg (100 mM) of 9-fluorenyl methoxycarbonyl hydrazine 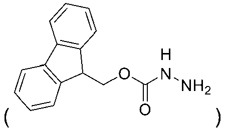 were added. The total volume was adjusted to 2 mL with 100 mM Tris-HCI buffer (pH 9.0), and the reaction was performed for 16 h at 37 °C through shaking. 500 µL of the system was taken, the reaction was terminated with 500 µL of acetonitrile, full shaking and well mixing were performed, then centrifugation was performed for 10 min at 12000 rpm, and detection (LC-MS) was performed to determine a conversion rate, as shown in Table 23 below.

**Table 23:**

| Enzyme number | Conversion rate | Enzyme number | Conversion rate | Enzyme number | Conversion rate | Enzyme number | Conversion rate |
|---|---|---|---|---|---|---|---|
| 1 | ++ | 78 | + | 155 | + | 232 | + |
| 2 | + | 79 | + | 156 | + | 233 | + |
| 3 | + | 80 | + | 157 | + | 234 | + |
| 4 | + | 81 | + | 158 | + | 235 | + |
| 5 | ++ | 82 | + | 159 | + | 236 | + |
| 6 | + | 83 | + | 160 | + | 237 | + |
| 7 | + | 84 | + | 161 | + | 238 | + |
| 8 | + | 85 | + | 162 | + | 239 | + |
| 9 | + | 86 | + | 163 | + | 240 | + |
| 10 | ++ | 87 | + | 164 | + | 241 | + |
| 11 | + | 88 | + | 165 | + | 242 | + |
| 12 | + | 89 | + | 166 | + | 243 | + |
| 13 | + | 90 | + | 167 | + | 244 | + |
| 14 | + | 91 | + | 168 | + | 245 | + |
| 15 | + | 92 | + | 169 | + | 246 | + |
| 16 | + | 93 | + | 170 | + | 247 | + |
| 17 | ++ | 94 | + | 171 | + | 248 | + |
| 18 | + | 95 | + | 172 | + | 249 | + |
| 19 | + | 96 | + | 173 | + | 250 | + |
| 20 | + | 97 | + | 174 | + | 251 | + |
| 21 | + | 98 | + | 175 | ++ | 252 | + |
| 22 | + | 99 | + | 176 | + | 253 | + |
| 23 | + | 100 | + | 177 | + | 254 | + |
| 24 | + | 101 | ++ | 178 | + | 255 | + |
| 25 | + | 102 | + | 179 | + | 256 | + |
| 26 | ++ | 103 | + | 180 | + | 257 | + |
| 27 | + | 104 | + | 181 | + | 258 | + |
| 28 | + | 105 | + | 182 | + | 259 | + |
| 29 | + | 106 | + | 183 | + | 260 | ++ |
| 30 | + | 107 | + | 184 | + | 261 | + |
| 31 | + | 108 | + | 185 | + | 262 | + |
| 32 | + | 109 | + | 186 | + | 263 | + |
| 33 | + | 110 | + | 187 | + | 264 | + |
| 34 | + | 111 | + | 188 | + | 265 | + |
| 35 | + | 112 | + | 189 | + | 266 | + |
| 36 | + | 113 | + | 190 | ++ | 267 | + |
| 37 | + | 114 | + | 191 | + | 268 | + |
| 38 | + | 115 | + | 192 | + | 269 | + |
| 39 | + | 116 | + | 193 | + | 270 | + |
| 40 | + | 117 | + | 194 | + | 271 | + |
| 41 | ++ | 118 | + | 195 | + | 272 | + |
| 42 | + | 119 | + | 196 | + | 273 | + |
| 43 | + | 120 | + | 197 | + | 274 | + |
| 44 | ++ | 121 | + | 198 | + | 275 | + |
| 45 | + | 122 | + | 199 | + | 276 | + |
| 46 | + | 123 | + | 200 | + | 277 | + |
| 47 | + | 124 | + | 201 | + | 278 | + |
| 48 | + | 125 | + | 202 | + | 279 | + |
| 49 | + | 126 | + | 203 | + | 280 | + |
| 50 | + | 127 | + | 204 | + | 281 | + |
| 51 | + | 128 | + | 205 | + | 282 | + |
| 52 | + | 129 | + | 206 | + | 283 | + |
| 53 | + | 130 | + | 207 | + | 284 | + |
| 54 | + | 131 | + | 208 | + | 285 | + |
| 55 | + | 132 | + | 209 | + | 286 | + |
| 56 | + | 133 | + | 210 | + | 287 | + |
| 57 | + | 134 | + | 211 | + | 288 | + |
| 58 | + | 135 | + | 212 | ++ | 289 | + |
| 59 | ++ | 136 | + | 213 | + | 290 | + |
| 60 | + | 137 | + | 214 | + | 291 | + |
| 61 | + | 138 | + | 215 | + | 292 | + |
| 62 | + | 139 | + | 216 | + | 293 | + |
| 63 | + | 140 | + | 217 | + | 294 | + |
| 64 | + | 141 | + | 218 | + | 295 | + |
| 65 | + | 142 | + | 219 | + | 296 | ++ |
| 66 | + | 143 | + | 220 | + | 297 | + |
| 67 | + | 144 | + | 221 | + | 298 | + |
| 68 | + | 145 | + | 222 | + | 299 | + |
| 69 | + | 146 | + | 223 | + | 300 | + |
| 70 | + | 147 | + | 224 | + | 301 | + |
| 71 | + | 148 | + | 225 | + | 302 | + |
| 72 | + | 149 | + | 226 | + | 303 | + |
| 73 | + | 150 | + | 227 | + | 304 | + |
| 74 | + | 151 | + | 228 | + | 305 | + |
| 75 | + | 152 | + | 229 | + | 306 | + |
| 76 | ++ | 153 | + | 230 | + | | |
| 77 | + | 154 | + | 231 | + | | |

### Embodiment 23:

16 different ketones were respectively selected and reacted with hydrazine hydrate under the action of Enzymes 1-20: in a 10 mL reaction bottle, imine reductase enzyme solutions of which enzyme numbers being 1-20 shown in Table 1 (a mass ratio of acetone to imine reductase in the enzyme solution was 1:10) were respectively added, then 1 wt of GDH (a mass ratio of one ketone), 21.6 mg of Glu, 1.33 mg of NAD⁺, 1.53 mg of NADP⁺, 20 mmol of ketone (dissolved in 10 µL of DMSO), and 20 mg (200 mM) of hydrazine hydrate were added. The total volume was adjusted to 2 mL with 100 mM Tris-HCI buffer (pH 9.0), and the reaction was performed for 16 h at 30 °C through shaking. 500 µL of the system was taken, the reaction was terminated with 500 µL of acetonitrile, full shaking and well mixing were performed, then centrifugation was performed for 10 min at 12000 rpm, and detection (HPLC or LC-MS) was performed; or 500 µL of the system was taken, the reaction was terminated with 100 µL of a 0.1 M sodium hydroxide solution, centrifugation was performed for 10 min at 12000 rpm, extraction was performed using ethyl acetate, and then detection (GC) was performed to determine a conversion rate, as shown in Tables 24-25.

**Table 24:**

| Enzyme | 1 | 2 | 3 | 4 | 5 | 6 | 7 | 8 | 9 | 10 |
|---|---|---|---|---|---|---|---|---|---|---|
| 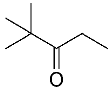 | ++ | + | + | ++ | + | + | + | + | ++ | + |
| 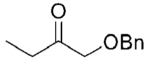 | + | +++ | ++ | + | + | + | ++ | ++ | + | +++ |
| 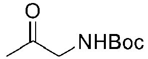 | + | + | ++ | + | ++ | ++ | + | ++ | ++ | ++ |
| 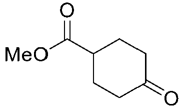 | +++ | ++++ | +++++ | + | +++++ | +++++ | ++++ | +++ | ++++ | +++++ |
| 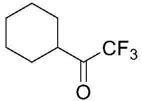 | +++ | ++ | ++++ | +++ | + | ++ | +++ | ++++ | +++ | + |
| 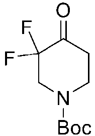 | ++ | +++ | +++ | + | ++ | + | + | ++ | + | +++ |
| 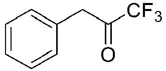 | + | +++ | + | + | ++ | ++ | + | + | ++ | +++ |
| 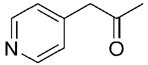 | + | + | + | ++ | +++ | + | + | ++ | + | + |
| 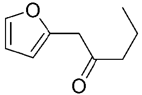 | + | + | + | ++ | + | + | + | ++ | + | + |
| 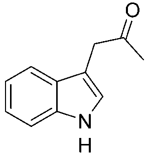 | ++ | +++ | + | + | +++ | ++ | + | + | + | ++ |
| 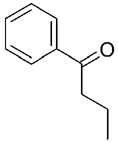 | + | + | ++ | + | + | + | + | + | ++ | + |
| 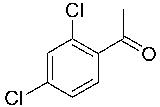 | +++ | +++ | ++++ | ++++ | + | ++++ | +++ | ++++ | ++++ | ++ |
| 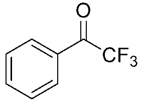 | +++ | ++++ | + | ++ | + | +++ | + | +++ | + | + |
| 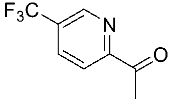 | + | + | ++ | ++++ | ++ | + | + | +++ | +++ | + |
| 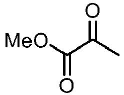 | + | + | ++ | ++ | + | + | + | + | ++ | ++ |
| 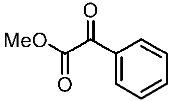 | + | + | + | ++ | + | + | + | + | ++ | + |

**Table 25:**

| Enzyme | 11 | 12 | 13 | 14 | 15 | 16 | 17 | 18 | 19 | 20 |
|---|---|---|---|---|---|---|---|---|---|---|
| 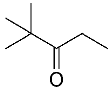 | + | + | + | + | +++ | + | +++ | + | ++++ | + |
| 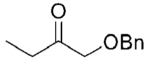 | + | ++ | ++ | + | + | + | ++ | ++ | + | + |
| 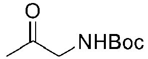 | ++ | + | +++ | + | + | + | + | +++ | + | + |
| 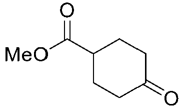 | ++++ | + | ++ | +++++ | + | ++++ | +++ | ++++ | +++++ | ++++ |
| 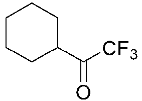 | +++ | + | + | ++ | +++ | + | +++ | + | +++ | ++ |
| 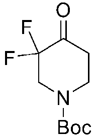 | +++ | ++++ | + | + | + | ++ | ++ | ++ | + | ++++ |
| 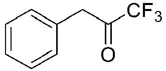 | + | + | ++ | ++ | + | ++ | + | + | +++ | ++ |
| 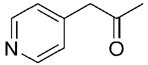 | + | ++ | + | + | +++ | ++ | +++ | + | + | + |
| 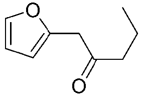 | + | + | + | + | ++ | + | + | + | + | + |
| 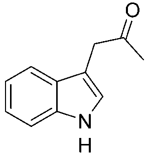 | + | + | + | +++ | + | + | ++ | + | + | ++ |
| 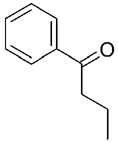 | + | +++ | + | + | + | + | + | + | ++ | + |
| 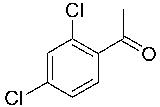 | ++ | +++ | ++++ | +++ | +++ | ++++ | ++++ | +++ | +++ | +++ |
| 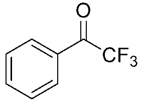 | ++++ | ++++ | + | + | + | + | +++ | + | ++++ | ++++ |
| 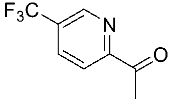 | + | ++ | ++ | +++ | ++++ | +++ | + | + | ++ | ++ |
| 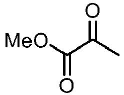 | + | + | + | + | +++ | + | ++ | + | +++ | + |
| 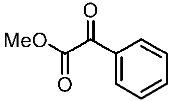 | + | + | + | + | + | + | ++ | ++ | + | + |

From the above description, it might be seen that the above embodiments of the present invention achieve the following technical effects: the method for generating, through the direct reaction between the ketone compound and the hydrazine compound catalyzed by the imine reductase, the hydrazine compound in which the N atom has no substituents or only one substituent is provided. The synthesis process is simple and economical, and various hydrazine compounds can be directly synthesized. Furthermore, the technical solution is mild in condition, easy to obtain raw materials and catalysts, low in cost, simple in operation, friendly in environment, and suitable for industrial-scale production.

The above are only the preferred embodiments of the present invention and are not intended to limit the present invention. For those skilled in the art, the present invention may have various modifications and variations. Any modifications, equivalent replacements, improvements and the like made within the spirit and principle of the present invention all fall within the scope of protection of the present invention.

## Claims

1. A method for preparing a hydrazine compound, comprising:
catalyzing the following reductive reaction of a substrate ketone compound shown in a formula I or formula III and a substrate hydrazine compound shown in a formula II with an imine reductase to obtain a product hydrazine compound; 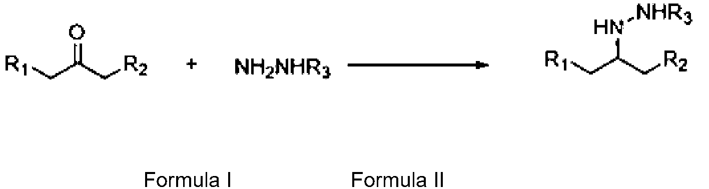 ; or 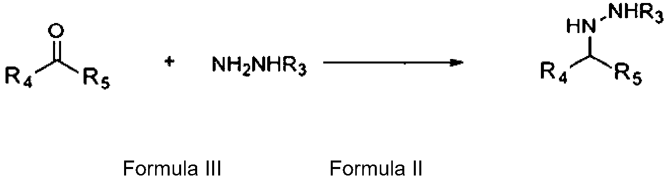
wherein R₁ and R₂ are each independently selected from hydrogen, cyano, , , pyridine, furan, 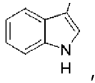 , a C₁-C₄ alkyl, a C₅-C₁₀ cycloalkyl or a C₅-C₁₀ substituted or unsubstituted heterocycloalkyl; or R₁ and R₂ are linked to form a C₃-C₈ substituted or unsubstituted cycloalkyl, a C₃-C₈ substituted or unsubstituted heterocycloalkyl or a C₉-C₁₅ substituted or unsubstituted benzocycloalkyl; R₄ and R₅ are each independently selected from trifluoromethyl, cyano, arylmethyl, methoxy, substituted or unsubstituted phenyl, a C₁-C₄ alkyl, a C₅-C₁₀ cycloalkyl, a C₅-C₁₀ substituted or unsubstituted heterocycloalkyl, 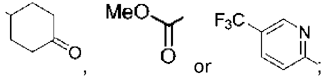
when the cycloalkyl, phenyl, or benzocycloalkyl is substituted, one or more H atoms of the cycloalkyl or the benzocycloalkyl are optionally substituted with a halogen atom or an alkyl;
one or more C atoms of the heterocycloalkyl are optionally substituted with N, O, P or S atom, and when the heterocycloalkyl is substituted, one or more H atoms of the heterocycloalkyl are optionally substituted with a halogen atom, benzyl, tert-butoxycarbonyl, or an alkyl;
R₃ is selected from hydrogen, a C₁-C₄ alkyl, a C₆-C₁₀ aryl, a C₁-C₄ hydroxyalkyl, substituted or unsubstituted C₁-C₄ acyl, substituted or unsubstituted C₆-C₁₀ arylmethyl, tert-butoxycarbonyl, benzyloxycarbonyl or 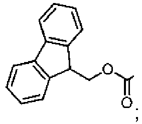 and
and when the acyl or arylmethyl is substituted, one or more H atoms of the acyl or arylmethyl are optionally substituted with a halogen atom, phenyl, or an alkyl.

2. The method of claim 1, **characterized in that**, the imine reductase is selected from any one of the imine reductases shown in Table 1.

3. The method of claim 1, **characterized in that**, R₁ and R₂ in the substrate ketone compound are each
independently selected from hydrogen, cyano, , 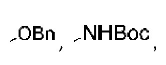 , pyridine, furan, 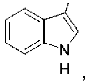
a C₁-C₄ alkyl, a C₅-C₆ cycloalkyl or a C₅-C₆ substituted or unsubstituted heterocycloalkyl;
or R₁ and R₂ are linked to form a C₅-C₆ substituted or unsubstituted cycloalkyl, a C₅-C₆ substituted or unsubstituted heterocycloalkyl or a C₉-C₁₃ substituted or unsubstituted benzocycloalkyl;
R₄ and R₅ are each independently selected from trifluoromethyl, cyano, arylmethyl, methoxy, substituted or unsubstituted phenyl, a C₁-C₄ alkyl, a C₅-C₆ cycloalkyl, a C₅-C₆ substituted or unsubstituted heterocycloalkyl, 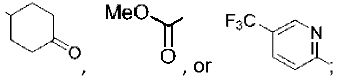
when the cycloalkyl, phenyl, or benzocycloalkyl is substituted, one or more H atoms of the cycloalkyl or benzocycloalkyl are optionally substituted with F atom, Cl atom, or methyl; and
one or more C atoms of the heterocycloalkyl are optionally substituted with N or O atoms, and when the heterocycloalkyl is substituted, one or more H atoms of the heterocycloalkyl are optionally substituted with Cl atom, F atom, benzyl, tert-butoxycarbonyl, or methyl .

4. The method of claim 1, **characterized in that**, R₃ in the substrate hydrazide compound is selected from hydrogen, a C₁-C₂ alkyl, a C₆ aryl, a C₁-C₂ hydroxyalkyl, benzyl, tert-butyloxycarbonyl, benzyloxycarbonyl,

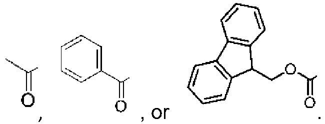

5. The method of claim 3, **characterized in that**, the substrate ketone compound comprises any one of the following:

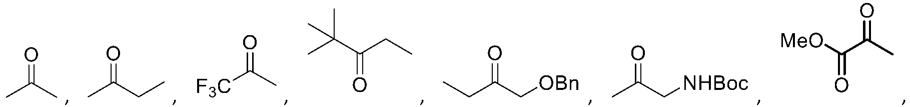

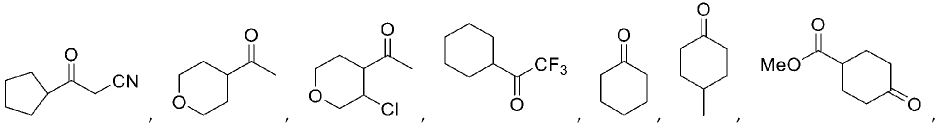

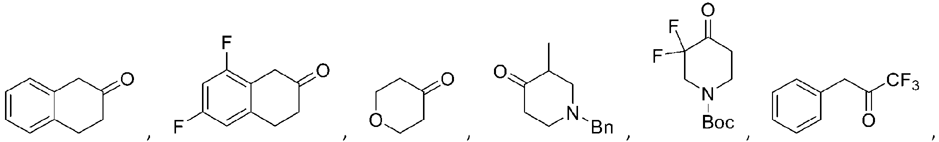

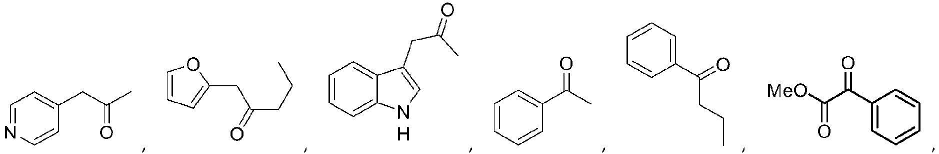

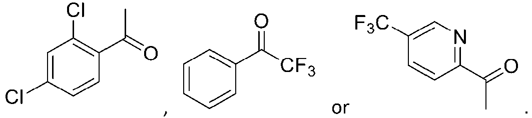

6. The method of claim 4, **characterized in that**, the substrate hydrazide compound comprises any one of the following: NH₂NH₂• H₂O, NH₂NHBoc , NH₂NHCbz , NH₂NH₂Bn ,

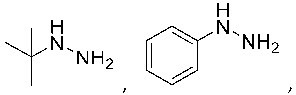

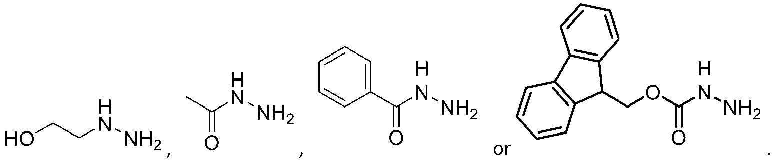

7. The method of claim 1, **characterized in that**, in a reaction system used for the reductive reaction, a molar ratio of the substrate ketone compound to the substrate hydrazine compound is 10-100: 50-200.

8. The method of claim 1, **characterized in that**, a mass ratio of the substrate ketone compound to the imine reductase is 1: 1-10.

9. The method of claim 1, **characterized in that**, a reaction system for the reductive reaction further comprises a glucose dehydrogenase, D-glucose, nicotinamide adenine dinucleotide, nicotinamide adenine dinucleotide phosphate and a buffer.
